# EUROPÄISCHE PATENTANMELDUNG

(11) **EP 4 763 519 A2**
(43) Veröffentlichungstag der Anmeldung: **24.06.2026**
(21) Anmeldenummer: 26176710.7
(22) Anmeldetag: 05.09.2019
(51) Int. Cl.: B33Y 80/00

(54) **VERFAHREN ZUM HERSTELLEN EINER ORTHOPÄDIETECHNISCHEN EINRICHTUNG**

(30) Priorität: 04.10.2018 DE 102018124516; 29.05.2019 DE 102019114458
(62) Teilanmeldung aus: 23206162.2
(71) Anmelder: Ottobock SE & Co. KGaA, 37115 Duderstadt (DE)
(72) Erfinder: BAUSE, Ingrid, 37345 Sonnenstein (DE); BERTELS, Thomas, 37115 Duderstadt (DE); BETZ, Sebastian, 37085 Göttingen (DE); BIERBAUM, Sarah, 37073 Göttingen (DE); BRÜNJES, Lukas, 37075 Göttingen (DE); ENGELBART, Hendryk, 37083 Göttingen (DE); FINKE, Lars, 37136 Landolfshausen (DE); HILLMANN, Martin, 37115 Duderstadt (DE); MÜLLER, Christian, 37589 Kalefeld (DE)
(74) Vertreter: Gramm, Lins & Partner Patent- und Rechtsanwälte PartGmbB

(57) **Zusammenfassung**

Die Erfindung betrifft Verfahren zum Herstellen eines Prothesenliners (2), der wie ein Strumpf über einen Amputationsstumpf gezogen werden kann, das dadurch gekennzeichnet ist, dass die orthopädietechnische Einrichtung zumindest teilweise mittels eines additiven Fertigungsverfahrens aus wenigstens einem Fertigungsmaterial (12) hergestellt wird, das in einem fließfähigen Zustand in ein Stützmaterial eingebracht wird und danach aushärtet, wobei das Fertigungsmaterial (12) aus wenigstens zwei Komponenten zusammengesetzt wird, wobei vorzugsweise ein Mischungsverhältnis der wenigstens zwei Komponenten während des additiven Fertigungsverfahrens einstellbar ist.

## Beschreibung

Die Erfindung betrifft ein Verfahren zum Herstellen einer orthopädietechnischen Einrichtung, insbesondere eines Prothesenliners. Die Erfindung betrifft zudem eine nach einem solchen Verfahren hergestellte oder herstellbare orthopädietechnische Einrichtung.

Unter einer orthopädietechnischen Einrichtung werden vorliegend insbesondere Orthesen und Prothesen sowie deren Bestandteile verstanden. Auch orthopädische Schuhe, Schuheinlagen und ähnliche Vorrichtungen werden als orthopädietechnische Einrichtung angesehen.

Orthopädietechnische Einrichtungen sind seit langem aus dem Stand der Technik bekannt und werden in einer Vielzahl unterschiedlicher Ausgestaltungen für unterschiedlichste Anwendungsfälle hergestellt und verkauft. Dabei wird eine Vielzahl unterschiedlicher Materialien verwendet, die den unterschiedlichen Anforderungsprofilen der jeweiligen orthopädietechnischen Einrichtung Rechnung tragen. So wird beispielsweise für eine Beinprothese ein Prothesenschaft hergestellt, in den ein Amputationsstumpf, beispielsweise ein Oberschenkelstumpf, eingeführt wird. Der Prothesenschaft muss eine ausreichende mechanische Stabilität und Festigkeit aufweisen, um den teilweise enormen Beanspruchungen Stand halten zu können und dem Träger der Prothese ein sicheres Standgefühl und gleichzeitig einen möglichst hohen Tragekomfort gewährleisten zu können. Zudem sollte der Prothesenschaft ein möglichst geringes Eigengewicht haben, wodurch der Komfort weiter erhöht wird. Heute werden derartige Prothesenschäfte zumeist aus einem Kohlefaserverbundwerkstoff hergestellt, der bei geringem Eigengewicht eine sehr hohe mechanische Festigkeit aufweist. Als Schnittstelle zwischen dem Prothesenschaft und dem Amputationsstumpf wird häufig ein Prothesenliner verwendet, der aus einem polsternden und elastischem Material, beispielsweise einem Silikon oder Polyurethan, hergestellt wurde. Dieser wird über den Amputationsstumpf wie ein Strumpf gezogen, bevor der Amputationsstumpf mit dem Prothesenliner in den Prothesenschaft eingeführt wird.

Während heute Prothesenschäfte in der Regel individuell für den Patienten hergestellt und in einem von mehreren bekannten Verfahren vom Amputationsstumpf abgeformt werden, wird der Prothesenliner in der Regel in Standardgrößen hergestellt, da er sich aufgrund seiner Elastizität an die individuellen Gegebenheiten anpasst.

Die Prothese selbst wird an einem Stumpf angeordnet und daran festgelegt. Zur Festlegung existieren unterschiedliche Systeme, ein Befestigungssystem sieht die sogenannte Vakuumschafttechnologie vor, bei der im angelegten Zustand das Volumen zwischen dem Stumpf und einer Prothesenschaftinnenwand evakuiert wird. Zur Abdichtung und zur Abpolsterung kann an dem Stumpf der Prothesenliner angeordnet sein, der in der Regel ein geschlossenes distales Ende und eine proximale Einstiegsöffnung aufweist und den Stumpf umgibt. Zwischen einer Außenseite des Prothesenliners und einer Innenseite des Prothesenschaftes wird durch das Einführen des mit dem Prothesenliner versorgten Stumpfes ein Volumen gebildet, das evakuiert wird, wodurch eine kraftschlüssige Verbindung zwischen dem Prothesenschaft und dem Prothesenliner entsteht. Der Prothesenliner haftet an dem Prothesenstumpf über Haftkräfte, sodass der Prothesenschaft und die an dem Prothesenschaft befestigten Komponenten an dem Stumpf des Patienten festgelegt sind. Um eine dauerhafte Befestigung des Prothesenschaftes zu erreichen, ist es notwendig, das Volumen zwischen dem Prothesenliner und dem Prothesenschaft gegenüber der Atmosphäre abzudichten. Dazu sind sogenannte Kappen oder Manschetten vorgesehen, die über den proximalen Rand des Prothesenschaftes gezogen werden und an der Außenseite des Prothesenliners oder des Stumpfes anliegen, sodass in den Spalt zwischen dem proximalen Rand des Prothesenschaftes und dem Prothesenliner oder dem Stumpf keine Luft eintreten kann. Alternativ zu einer Manschette oder auch Kappe können Dichtlippen an der Außenseite des Liners oder an der Innenseite des Prothesenschaftes angeordnet oder festgelegt sein, um eine Abdichtung eines Volumens zu bewirken.

Der Prothesenschaft besteht in der Regel aus einem formstabilen Material, um eine ausreichende Stabilität und Festigkeit zu haben, um weitere Prothesenkomponenten daran anzuordnen und eine Stützfunktion für die Weichteilkomponenten des Amputationsstumpfes bereitzustellen. Der proximale Rand des Prothesenschaftes ist dabei möglichst hochgezogen ausgebildet, um den Amputationsstumpf sicher aufnehmen zu können. Bei einem Unterschenkelschaft ragt der proximale Rand beispielsweise medial und lateral bis zu den Kniekondylen und ist im Schienbeinbereich und im Kniekehlenbereich tief ausgeschnitten. Eine ähnliche Konstruktion ergibt sich bei einem Unterarmschaft. Bei einem Oberschenkelschaft ist eine laterale Erhöhung ausgebildet, um eine seitliche Stabilität bereitzustellen.

Der Erfindung liegt die Aufgabe zugrunde, für eine Vielzahl unterschiedlicher orthopädietechnischer Einrichtungen ein verbessertes Herstellverfahren anzugeben.

Die Erfindung löst die gestellte Aufgabe durch ein Verfahren zum Herstellen einer orthopädietechnischen Einrichtung, insbesondere eines Prothesenliners, das sich dadurch auszeichnet, dass die orthopädietechnische Einrichtung zumindest teilweise mittels eines additiven Fertigungsverfahrens aus wenigstens einem Fertigungsmaterial hergestellt wird, das in einem fließfähigen Verarbeitungszustand in ein Stützmaterial eingebracht wird und danach aushärtet.

Zu den additiven Fertigungsverfahren gehören neben verschiedenen 3-D Druckverfahren auch ein beispielsweise von dem MIT entwickeltes dreidimensionales Druckverfahren, das unter dem Stichwort "Rapid Liquid Printing" veröffentlicht wurde. Dabei wird der herzustellende Gegenstand in einem Behälter erzeugt, der mit einer Gelsuspension oder einem anderen Material gefüllt ist, das mit dem Fertigungsmaterial nicht chemisch reagiert, sondern ausschließlich zur mechanischen Stützung des Fertigungsmaterials dient, solange dieses noch nicht ausgehärtet oder ausreichend vernetzt ist. Die Gelsuspension ist in diesem Fall das Stützmaterial. Bei all diesen Verfahren wird das Fertigungsmaterial in fließfähiger Form, beispielsweise flüssig verarbeitet. Beim Beispiel des "Rapid Liquid Printing" wird das Fertigungsmaterial in flüssiger oder gelförmiger Form, die vorliegend beide als fließfähig angesehen werden, mittels einer Positioniereinrichtung, beispielsweise einer Düse, die dreidimensional bewegbar ist, an den gewünschten Positionen in die Gelsuspension eingebracht. Aufgrund der Dichteverhältnisse zwischen dem Fertigungsmaterial und dem Stützmaterial der Gelsuspension sowie der hohen Viskosität des Stützmaterials verbleibt das eingebrachte Fertigungsmaterial an der jeweiligen Position. Auf diese Weise können dreidimensionale Gegenstände "gedruckt" werden, indem das Fertigungsmaterial an den gewünschten Positionen in der gewünschten Form in die Gelsuspension eingebracht wird und anschließend dort vernetzt, erstarrt oder aushärtet. Wird nachfolgend von Aushärten gesprochen, ist damit auch das Vernetzen oder ein Anderweitiges Reagieren oder Verändern von Eigenschaften des Fertigungsmaterials umfasst, die zu einer Erhöhung der Formstabilität oder der Erreichung eines gewünschten Zustandes der orthopädietechnischen Einrichtung oder eines Bauteils führt. Insbesondere bei flexiblen oder elastischen Materialien bleibt die Flexibilität oder Elastizität nach dem Aushärten weiterhin erhalten. Das Vernetzen wird als Aushärten im Sinne der Erfindung verstanden. Der Vorteil gegenüber herkömmlichen 3-D Druckverfahren liegt unter anderem in der Vielzahl möglicher Fertigungsmaterialien, inklusive am Markt verfügbarer, bei Raumtemperatur vernetzenden Silikone. Ein weiterer Vorteil liegt darin, dass es aufgrund des Prozesses unter Zuhilfenahme der Gelsuspension möglich ist, dreidimensionale Objekte direkt in einem Arbeitsraum des Druckers zu positionieren und nicht schichtweise aufbauen zu müssen. Darüber hinaus ermöglicht das Verfahren hohe Herstellungsgeschwindigkeiten und dadurch geringe Herstellungskosten.

Fertigungsmaterialien sind beispielsweise Silikone, Polyurethane aber auch thermoplastische Werkstoffe, Gießharze oder andere Kunststoffe. Für ein Fertigungsmaterial ist lediglich wichtig, dass es in einer applizierbaren Form, also fließfähig, beispielsweise flüssig oder auf andere Weise spritzbar, verarbeitet werden und aushärten kann.

Auf diese Weise lassen sich beispielsweise Prothesenliner, Prothesenhandschuhe, Einlegesohlen und andere orthopädietechnische Einrichtungen aus herkömmlichem Silikon einfach, schnell und gegebenenfalls individuell geformt herstellen. Werden thermoplastische Werkstoffe verwendet, kann die nach dem Aushärten vorliegende mechanische Stabilität und Härte ausreichend sein, um durch dieses Verfahren beispielsweise Prothesenschäfte, Gelenkprotektoren oder Versteifungselemente wie beispielsweise Schienen für orthopädietechnische Einrichtungen auf diese Weise herzustellen. Auch Prothesenverkleidungen und Prothesenkosmetiken können auf diese Weise hergestellt werden. Zudem können auch Probeprothesen, beispielsweise Probeschäfte oder Probekosmetiken hergestellt werden.

Durch die Verwendung des additiven Fertigungsverfahrens ist es besonders einfach möglich, beispielsweise eine Prothesenkosmetik, insbesondere eine Fußkosmetik, eine Knöchelmanschette oder beispielsweise eine individuell gefertigte Patella oder eine individuelle Kniekappe mit oder ohne Schaftanschluss herzustellen. Eine solche orthopädietechnische Einrichtung kann auf diese Weise beispielsweise wasserdicht und/oder mit einer Funktionalisierung, beispielsweise mit einer erhöhten Beweglichkeit durch eine Faltenbalkstruktur im Handgelenksbereich einer Prothesenhand ausgebildet werden.

In einer bevorzugten Ausgestaltung wird das Fertigungsmaterial während des Aushärtens von dem Stützmaterial gestützt und/oder in seiner Position in einem Arbeitsraum gehalten. Vorzugsweise handelt es sich um ein selbsthärtendes oder durch Temperaturerhöhung härtbares Material. Bei der Verwendung eines selbsthärtenden Materials ist von Vorteil, dass keine Einwirkung mittels anderer Härter, beispielsweise elektromagnetischer Strahlung oder chemischer Zusätze, nötig ist. Diese Vorteile werden auch durch die Verwendung eines durch Temperaturerhöhung härtbaren Materials erreicht, das beispielsweise durch die Applikation von Wärmestrahlung aushärtet.

Zusätzlich zu dem Fertigungsmaterial, das im fließfähigen Zustand in das Stützmaterial eingebracht wird, können auch Fasern, insbesondere Endlosfasern, mit eingebracht werden. Diese können gleichzeitig mit dem Fertigungsmaterial appliziert und insbesondree von diesem umschlossen werden. Die Fasern beinhalten oder sind vorzugsweise Carbonfasern, die das herzustellende Bauteil verstärken.

Bevorzugt wird das Fertigungsmaterial aus wenigstens zwei Komponenten zusammengesetzt. Vorzugsweise ist ein Mischungsverhältnis der wenigstens zwei Komponenten zueinander einstellbar. Besonders bevorzugt ist das Mischungsverhältnis während der Durchführung des additiven Fertigungsverfahrens einstellbar. Durch diese Ausgestaltung kann beispielsweise das Fertigungsmaterial in mehreren Komponenten, die jede für sich in einem fließfähigen Zustand ist, appliziert werden. Jede der Komponenten für sich ist dabei vorzugsweise so ausgebildet, dass es nicht oder nur sehr langsam zu einer Aushärtung kommt. Kommen die beiden Komponenten jedoch in Kontakt miteinander, kann eine chemische Reaktion hervorgerufen werden, durch die das eigentliche Fertigungsmaterial entsteht, das bevorzugt schnell aushärtet. Durch eine Veränderung des Mischungsverhältnisses lassen sich die physikalischen und/oder chemischen Eigenschaften des auf diese Weise hergestellten Fertigungsmaterials beeinflussen, sodass auch der auf diese Weise hergestellte Teil der orthopädietechnischen Einrichtung nahezu stufenlos unterschiedliche physikalische Eigenschaften aufweist und entsprechende Gradienten beinhalten kann.

In einer bevorzugten Ausgestaltung ist das Fertigungsmaterial beispielsweise so ausgebildet, dass es nach dem Aushärten eine Shore-Härte aufweist, deren Wert von dem Mischungsverhältnis abhängt. Auf diese Weise lassen sich Bereiche mit großer Härte und Bereiche mit geringerer Härte in einem Verfahrensschritt direkt nebeneinander und aneinander einstückig herstellen, ohne dass die Vorrichtung verändert oder das verwendete Material geändert werden muss. Es ist ausreichend, lediglich das Mischungsverhältnis der wenigstens zwei Komponenten zu verändern, was während des additiven Fertigungsverfahrens geschehen kann.

Auf diese Weise kann vorzugsweise eine kontinuierliche Variation eines Materialparameters oder einer physikalischen Eigenschaft des Fertigungsmaterials durch eine kontinuierliche Variation eines Mischungsverhältnisses erreicht werden. Denkbar ist beispielsweise ein Unterschenkelliner, der im Bereich der Kniekehle deutlich elastischer ausgeführt ist als dies in anderen Bereichen der Fall ist. Alternativ oder zusätzlich dazu lassen sich Bauteile mit komplexen Gradienten hinsichtlich der Wandstärken realisieren, die mit Fertigungsverfahren aus dem Stand der Technik und dem dafür beispielsweise benötigten Formenbau nicht oder nur mit großem Aufwand hergestellt werden können.

In einer bevorzugten Ausgestaltung werden in dem additiven Fertigungsverfahren wenigstens zwei unterschiedliche Fertigungsmaterialien verwendet. Dies geschieht vorteilhafterweise gleichzeitig. Die beiden unterschiedlichen Fertigungsmaterialien unterscheiden sich dabei in wenigstens einer Eigenschaft. So kann beispielsweise das Fertigungsmaterial in unterschiedlichen Farben verwendet werden, um gewünschte optische Effekte und ästhetische Eindrücke zu erzeugen. Durch die Verwendung von beispielsweise einem eingefärbten Fertigungsmaterial lassen sich auch Markierungen an oder in der zu fertigenden orthopädietechnischen Einrichtung oder zumindest an einem Bauteil der orthopädietechnischen Einrichtung, das mittels des additiven Fertigungsverfahrens wenigstens teilweise hergestellt wird, anbringen. Dies ist beispielsweise dann sinnvoll, wenn die orthopädietechnische Einrichtung in einer bestimmten Orientierung am Körper des Patienten getragen werden soll. Verfügt beispielsweise ein Prothesenliner über eingearbeitete Elektroden, um beispielsweise myoelektrische Signale vom Amputationsstumpf abzunehmen oder die Muskeln des Amputationsstumpfes mit elektrischen Signalen zu stimulieren oder zur Plasmabehandlung, ist es wichtig, dass die Elektroden reproduzierbar an der richtigen Stelle des jeweiligen Amputationsstumpfes angeordnet werden. Dies gilt selbstverständlich auch für Elektroden, die an einer Orthese angeordnet sind, die über ein noch vorhandenes Körperteil des Patienten gezogen wird. Durch die Verwendung von Markierungen kann es in diesen Fällen den Patienten erleichtert werden, die orthopädietechnische Einrichtung, beispielsweise den Prothesenliner, in der richtigen Orientierung am Körperteil anzulegen. Diese Markierungen lassen sich mit dem Verfahren gemäß diesem Ausführungsbeispiel der vorliegenden Erfindung besonders einfach dadurch herstellen, dass an der Stelle, an der die Markierung angeordnet werden soll, das Fertigungsmaterial des additiven Fertigungsverfahrens in einer anderen Farbe eingefärbt verwendet wird.

Durch die Verwendung zweier unterschiedlich eingefärbter Fertigungsmaterialien lässt sich zudem ein Verschleißindikator oder ein Beschädigungsindikator herstellen. Ist beispielsweise bei einer intakten orthopädietechnischen Einrichtung nur das Fertigungsmaterial einer ersten Farbe von außen sichtbar, wird durch das Erscheinen einer zweiten Farbe ein deutliches Signal dafür gegeben, dass beispielsweise ein Verschleißteil ausgetauscht werden muss oder die orthopädietechnische Einrichtung einen Defekt aufweist.

Die wenigstens zwei unterschiedlichen Fertigungsmaterialien unterscheiden sich vorzugsweise in ihrer elektrischen Leitfähigkeit. Während beispielsweise ein herkömmliches Silikon oder Polyurethan elektrisch isolierend ist, kann es durch die Zugabe von entsprechenden Additiven, beispielsweise Rußpartikel oder Metallspänen, elektrisch leitfähig gemacht werden. Aus der nicht vorveröffentlichen DE 10 2017 126 465 sind entsprechende orthopädietechnische Einrichtungen bekannt, deren Grundkörper aus einem elektrisch isolierenden Material besteht, der beispielsweise aus dem ersten Fertigungsmaterial mittels des additiven Fertigungsverfahrens hergestellt werden kann. Darin befindet sich ein elektrischer Leiter mit einem Kern aus einem elektrisch leitfähigen Elastomer, der eine elektrisch isolierende Beschichtung aufweist. Diese elektrisch isolierende Beschichtung in Form beispielsweise einer Parylen-Beschichtung dient als Haftvermittler zwischen dem Leiter aus dem elektrisch leitfähigen Elastomer und dem Grundkörpermaterial. In der Ausgestaltung der vorliegenden Erfindung kann jedoch das Grundkörpermaterial in Form des ersten Fertigungsmaterials und das elektrisch leitfähige Elastomer-Material des elektrischen Leiters in Form eines zweiten Fertigungsmaterials vorzugsweise gleichzeitig verarbeitet werden. In einer bevorzugten Ausgestaltung handelt es sich sowohl bei dem Material des elektrischen Leiters als auch bei dem Grundkörpermaterial, also bei beiden hier beschriebenen Fertigungsmaterialien, jeweils um ein Silikon. Auf diese Weise werden die elastischen Eigenschaften des ersten Fertigungsmaterials, das im beschriebenen Ausführungsbeispiel den Grundkörper bildet, nicht durch den elektrischen Leiter beeinflusst. Eine Haftvermittlerschicht ist unnötig, da beide Fertigungsmaterialien gleichzeitig aushärten. Auf diese Weise kommt es zu einer optimalen Verbindung zwischen den beiden Fertigungsmaterialien.

Durch das hier beschriebene Verfahren kann folglich wenigstens ein elektrischer Leiter, bevorzugt jedoch mehr als einer der elektrischen Leiter, in dem Fertigungsmaterial der orthopädietechnischen Einrichtung angeordnet werden.

Alternativ oder zusätzlich dazu unterscheiden sich die verwendeten Fertigungsmaterialien nach dem Aushärten beispielsweise in ihrer Härte und/oder in ihrer Elastizität. Die unterschiedlichen Fertigungsmaterialien können an unterschiedlichen Stellen der orthopädietechnischen Einrichtung eingesetzt werden, sodass beispielsweise bei einem Prothesenliner die Teile, an denen ein Knochen sehr nah an der Außenseite des Amputationsstumpfes positioniert ist und die einer besonderen Polsterung bedürfen, mit einem besonders weichen und polsternden Material überdeckt werden können. Handelt es sich bei dem Prothesenliner beispielsweise um einen Prothesenliner für Unterschenkelamputierte, kann der Bereich der Kniekehle mit einem besonders elastischen Fertigungsmaterial hergestellt werden, um den starken mechanischen Belastungen durch die vielfache Dehnung gerecht zu werden. Weiterhin ist es möglich, durch die Verwendung eines wenig elastischen Fertigungsmaterials innerhalb eines höher elastischen Fertigungsmaterials Versteifungen zu erzeugen, um beispielsweise die Längssteifigkeit eines Prothesenliners zu erhöhen, gleichzeitig aber die Querdehnbarkeit beizubehalten. Zudem ist es möglich, durch das additive Fertigungsverfahren eine auxetische Struktur zu schaffen, wie sie beispielsweise in der DE 10 2017 106 903 beschrieben und für Prothesenliner verwendet wird. Auxetische Materialien haben negative Poissonzahlen. Dies bedeutet, dass eine Verlängerung des Materials in einer Richtung, anders als bei herkömmlichen Materialien, nicht zu einer Verkürzung in einer senkrecht auf dieser Richtung stehenden zweiten Richtung führt, sondern auch in dieser Richtung eine Verlängerung zur Folge hat. Insbesondere zweidimensional auxetische Materialien können in vorteilhafter Weise in Linern eingesetzt werden. Die dafür notwendigen Strukturen lassen sich durch die hier beschriebenen Fertigungsverfahren besonders einfach und kostengünstig auch in großer Stückzahl herstellen.

Werden die unterschiedlichen Fertigungsmaterialien für unterschiedliche Bereiche der orthopädietechnischen Einrichtung oder eines Bauteils der orthopädietechnischen Einrichtung, das durch das additive Fertigungsverfahren hergestellt wird, verwendet, können die unterschiedlichen Fertigungsmaterialien auch nacheinander verwendet werden. Eine Vorrichtung, mit der das additive Fertigungsverfahren ausgeführt wird, verfügt in diesem Fall vorzugsweise über mehrere Austrittsöffnungen, die vorzugsweise mit unterschiedlichen Fertigungsmaterial-Behältern verbunden sind, sodass die unterschiedlichen Fertigungsmaterialien direkt nacheinander und/oder gleichzeitig verwendet werden können.

Vorzugsweise wird das wenigstens eine Fertigungsmaterial während des additiven Fertigungsverfahrens mit einem separat gefertigten Bauteil der orthopädietechnischen Einrichtung verbunden. Dazu wird das separat gefertigte Bauteil, das beispielsweise eine Verbindungskappe, ein aus einem anderen Material, beispielsweise Metall, gefertigter Verbindungsadapter, eine Elektrode, eine Batteriehalterung oder ein sonstiges Element sein kann, in der Vorrichtung und innerhalb des Stützmaterials angeordnet, in der das additive Fertigungsverfahren durchgeführt wird. Bei dem Verfahren des "Rapid Liquid Printings" wird das jeweilige separat gefertigte Bauteil folglich innerhalb der Gelsuspension angeordnet. Das Fertigungsmaterial für das additive Fertigungsverfahren wird dann während des additiven Fertigungsverfahrens so positioniert, dass es mit dem jeweils separat gefertigten Bauteil in Kontakt kommt, sodass es beim Aushärten des Fertigungsmaterials zu einer stoffschlüssigen Verbindung zwischen den separat gefertigten Bauteil und dem Fertigungsmaterial kommt. Selbstverständlich kann das Fertigungsmaterial auch so angeordnet werden, dass es alternativ oder zusätzlich dazu zu einer formschlüssigen Verbindung kommt, nachdem das Fertigungsmaterial ausgehärtet ist. Alternativ oder zusätzlich ist es möglich, separat gefertigte Bauteile in das bereits gedruckte aber noch nicht ausgehärtete Fertigungsmaterial einzubringen. Es können so beispielsweise, nachdem ein Prothesenliner nach dem beschriebenen Verfahren hergestellt wurde, in dessen Wandungen Kabel oder Versteifungsfäden oder ähnliches positioniert werden, die sich anschließend stoff- und oder formschlüssig mit dem aushärtenden Fertigungsmaterial verbinden.

Ein Prothesenliner verfügt im distalen Bereich oftmals über eine sogenannte Linerkappe, mit der eine mechanische Verbindung zum Prothesenschaft hergestellt werden kann. Auch elektronische Bauteile, Sensoren, pneumatische, hydraulische oder sonstige Elemente können in der Linerkappe angeordnet sein. Die Linerkappe wird separat gefertigt und kann als separat gefertigtes Bauteil während des additiven Fertigungsverfahrens mit dem Fertigungsmaterial verbunden werden. Gleiches gilt für elektronische Bauteile, beispielsweise Sensoren, Elektroden oder sonstige Elemente. Auch diese sind als separat gefertigte Bauteile zu betrachten.

In einer bevorzugten Ausgestaltung des Verfahrens wird in das Stützmaterial wenigstens ein Gegenstand eingebracht, an den das Fertigungsmaterial während des additiven Fertigungsverfahrens angedruckt wird. Das Fertigungsmaterial wird folglich so im fließfähigen Zustand in das Stützmaterial eingebracht, dass es mit dem in das Stützmaterial eingebrachten Gegenstand in Kontakt kommt. Beim Aushärten des Fertigungsmaterials entsteht dabei vorzugsweise eine stoffschlüssige und/oder formschlüssige Verbindung zwischen dem eingebrachten Gegenstand und dem ausgehärteten Fertigungsmaterial. So ist es beispielsweise möglich, einen aus einem in elastischen Material hergestellten Teil einer orthopädietechnischen Einrichtung, beispielsweise einen Prothesenschaft, in das Stützmaterial einzubringen und anschließend Polster und/oder Kissen im additiven Fertigungsverfahren anzudrucken. Die Polster oder Kissen werden dabei zumindest teilweise, vorzugsweise jedoch vollständig aus dem Fertigungsmaterial hergestellt. Dieses wird im fließfähigen Zustand so in das Stützmaterial eingebracht, dass es mit dem Prothesenschaft, der ebenfalls in dem Stützmaterial angeordnet ist, in Kontakt kommt.

Dieses Verfahren ist insbesondere dann von Vorteil, wenn eine genaue Passform zwischen zwei Bauteilen nötig oder gewünscht ist. So kann beispielsweise eine Prothesenkosmetik, also die die eigentliche Prothese umhüllende Kunststoffhülle, direkt an die Prothese angedruckt werden. So kann beispielsweise eine Prothesenhand oder eine andere orthopädietechnische Einrichtung in das Stützmaterial eingebracht, beispielsweise eingetaucht werden. Der im additiven Fertigungsverfahren herzustellende Teil der orthopädietechnischen Einrichtung kann dann direkt an das eingebrachte Bauteil angedruckt werden. Im Zuge des additiven Fertigungsverfahrens können auch Funktionalitäten, beispielsweise individuelle Dämpfungseigenschaften, hergestellt werden. Ein Prothesenfuß und/oder eine Prothesenfußhülle, die den eigentlichen Prothesenfuß umgibt, können so beispielsweise an unterschiedlichen Stellen aus unterschiedlichen Fertigungsmaterialien hergestellt werden, sodass sich unterschiedliche Dämpfungseigenschaften ergeben.

Zudem verfügen insbesondere Prothesenliner, jedoch nicht nur diese orthopädietechnische Einrichtungen, oftmals über eine Textillage oder andere textile Bauteile. Ist dies der Fall, kann auch dieses textile Element als separat gefertigtes Bauteil angesehen und während des additiven Fertigungsverfahrens mit dem Fertigungsmaterial verbunden werden.

Orthesen dienen oftmals dazu, einen Druck auf bestimmte Körperstellen auszuüben, um so beispielsweise Gelenke zu stützen oder Bänder und Sehnen zu entlasten. Dazu werden bevorzugt Pelotten verwendet, die oftmals aus einem Silikon hergestellt sind und durch die Orthese so am Körper des Trägers angeordnet werden, dass beispielsweise nach dem Schließen eines zusätzlichen Gurtes ein Druck auf die gewünschte Stelle ausgeübt wird. Auch eine solche Pelotte kann durch ein Verfahren der hier beschriebenen Art aus einem Fertigungsmaterial hergestellt werden, wobei das Fertigungsmaterial bevorzugt mit dem separat gefertigten Bauteil, also dem beispielsweise aus einem Textil bestehenden Grundkörper der Orthese, verbunden wird.

Wie bereits dargelegt, können Bauteile und Elemente, die mit dem Fertigungsmaterial in Verbindung stehen sollen, bereits in das Stützmaterial eingebracht sein, wenn das Fertigungsmaterial im fließfähigen Zustand aufgebracht wird. Dies ist jedoch bei flexiblen Elementen, wie beispielsweise Textilien, nur eingeschränkt möglich, da diese keine oder nur eine geringe Formstabilität aufweisen. Es hat sich daher als vorteilhaft herausgestellt, beispielsweise einen Liner oder ein Teil einer anderen orthopädietechnischen Einrichtung als geschlossenes Volumen herzustellen. Im Innern dieses Volumens befindet sich dann ein Stützmaterial. Auf diesen so geschlossen ausgebildeten Liner kann nun aufgrund des Widerstandes, der durch das eingeschlossene Stützmaterial hervorgerufen wird, besonders einfach eine Textillage aufgebracht werden, ohne dass eine Form oder ein Werkzeug hergestellt werden müsste. Ist die Textillage aufgebracht, kann der Liner am proximalen Ende geöffnet und das enthaltene Stützmaterial entfernt werden. Die aufgebrachte Textillage kann vorher beispielsweise aufgeklebt werden. Zusätzlich oder alternativ zudem Aufbringen von Textilien oder anderen Materialien ist es in bestimmten Situationen vorteilhaft, ein weiteres Bauteil, beispielsweise einen Prothesenschaft, direkt auf dem Liner abzuformen. Auch dafür ist es von Vorteil, wenn der Liner einer Belastung einen Widerstand entgegensetzen kann, was insbesondere auf die oben genannten Weisen erreichbar ist.

Um den Effekt des eingeschlossenen Stützmaterials zu verstärken, kann der durch das Stützmaterial hervorgerufene Widerstand insbesondere bei Wasser basiertem Stützmaterial dadurch verstärkt werden, dass der geschlossen ausgebildete Teil der orthopädietechnischen Einrichtung, in dem das Stützmaterial eingeschlossen ist, eingefroren wird. Dadurch wird das Stützmaterial hart und kann die beim Aufbringen und insbesondere Aufkleben der Textillage wirkenden Kräfte abfangen, ohne dass der nun gekühlte und gefrorene Liner, der vorzugsweise noch immer geschlossen ist, sich verformt. Alternativ zu der geschlossen ausgebildeten Variante des im additiven Fertigungsverfahrens hergestellten Teiles der orthopädietechnischen Einrichtung kann auch ein Zulauf und/oder Ablauf, insbesondere mit oder ohne einem Ventil vorhanden sein, durch den beispielsweise das enthaltene Stützmaterial aus dem ansonsten geschlossenen Teil der orthopädietechnischen Einrichtung entfernt werden kann. Wird eine erhöhte Formstabilität dieses Teils gewünscht, kann beispielsweise durch einen Schlauch, der mit dem Zulauf verbunden ist, ein Medium, beispielsweise Luft, in den ansonsten geschlossenen Teil der orthopädietechnischen Einrichtung eingebracht und auf diese Weise der benötigte Druck aufgebaut werden. Nachdem die Textillage aufgebracht wurde, kann der geschlossene Teil der orthopädietechnischen Einrichtung geöffnet und gegebenenfalls enthaltenes Stützmaterial entfernt werden.

Alternativ oder zusätzlich dazu können Versteifungselemente in den ansonsten geschlossenen Teil der orthopädietechnischen Einrichtung eingebracht werden, die ausgebildet sind, durch ein Entfernen oder Abführen von Fluid aus dem Volumen insbesondere miteinander in Kontakt gebracht zu werden oder zumindest ihren Kontakt durch das Entfernen oder Abführen zu verstärken. In einem sehr einfachen Ausführungsbeispiel kann es sich dabei um ein Granulat, beispielsweise Sand, handeln. Wird in diesem Fall beispielsweise Luft aus dem mit den Versteifungselementen, also dem Granulat, befüllten Volumen entfernt oder abgeführt, kommen die einzelnen Versteifungselemente in Kontakt oder verstärken diesen Kontakt mit einander. Dadurch kann eine rigide Form ausgebildet werden, die die umgebende und/oder enthaltene orthopädietechnische Einrichtung stützt.

In dem additiven Fertigungsverfahren kann die Wandstärke der orthopädietechnischen Einrichtung kontinuierlich oder in diskreten Schritten variiert werden, sodass mindestens eine Ausbuchtung, Vertiefung, Aufdickung, Verjüngung und/oder ein Hinterschnitt erzeugt wird.

Vorteilhafterweise wird mit dem additiven Fertigungsverfahren eine orthopädietechnische Einrichtung und/oder ein Bauteil mit wenigstens einem Hohlraum erzeugt. Grundsätzlich ist dabei vorgesehen, einen Hohlraum zu erzeugen, indem ein Bereich der Gelsuspension mit Fertigungsmaterial umdruckt wird. Alternativ oder zusätzlich lässt sich ein Hohlraum aber auch herstellen, indem zunächst das Fertigungsmaterial als Vollmaterial ausgebracht wird, in das anschließend in noch flüssigem Zustand ein Hilfsmaterial eingebracht wird, das den entsprechenden Hohlraum formt. Das Hilfsmaterial kann dabei ein Gas oder eine Flüssigkeit, zum Beispiel auch die verwendete Gelsuspension, sein und nach Fertigstellung des Bauteiles in diesem verbleiben, um beispielsweise ein Luftpolster herzustellen, oder aus dem ausgehärteten Bauteil wieder entfernt werden. Der Hohlraum, insbesondere ein wie oben beschrieben hergestellter Hohlraum, kann unterschiedliche Eigenschaften aufweisen und verschiedenste Aufgaben erfüllen. Dieser Hohlraum kann ein geschlossener Hohlraum sein oder eine Öffnung oder einen Durchbruch aufweisen. So kann beispielsweise eine durchbrochene Struktur erzeugt werden, die beispielsweise wabenförmig ausgebildet ist und der mechanischen Festigkeit dient.

Handelt es sich beim wenigstens einen Hohlraum jedoch um einen abgeschlossenen Hohlraum, kann dieser beispielsweise als Luftpolster verwendet werden, um besonders empfindliche Stellen des Körpers des Trägers der orthopädietechnischen Einrichtung zu polstern und auf diese Weise den Tragekomfort zu erhöhen. Auf diese Weise hergestellte Luftpolster können beispielsweise auch mit einem Anschluss ausgebildet sein, um Luft in das Luftpolster hinein zu pumpen oder aus ihm herauszulassen. Damit kann der Druck im Innern des Luftpolsters und gegebenenfalls auch die Ausdehnung des Luftpolsters eingestellt und an die individuellen Bedürfnisse angepasst werden. So kann beispielsweise einer Volumenschwankungen eines Amputationsstumpfes Rechnung getragen werden. Befindet sich an einer dem Körper zugewandten Seite des Luftpolsters beispielsweise eine Elektrode, kann der Anpressdruck, mit dem die Elektrode gegen die Haut des Trägers gedrückt wird, durch ein solches aufblasbaren Luftpolster eingestellt und optimiert werden.

Mit einem derartigen Luftpolster kann auch großflächig und/oder zonenabhängig Druck ausgeübt werden, der beispielsweise zur Versorgung von Narbengewebe bei der Verbrennungsversorgung notwendig und von Vorteil ist. Hergestellte Hohlräume können auch in Form von Kanälen vorhanden sein, die beispielsweise Kühlmittel beinhalten, um einen mit der orthopädietechnischen Einrichtung in Kontakt stehenden Körperteil zu kühlen. Auch zum Medikamententransport können derartige Kanäle, die als Hohlräume im additiven Fertigungsverfahren aus dem Fertigungsmaterial hergestellt werden, verwendet werden.

Alternativ oder zusätzlich dazu kann der wenigstens eine Hohlraum während des additiven Fertigungsverfahrens mit wenigstens einem Füllmaterial zumindest teilweise, bevorzugt jedoch vollständig, gefüllt werden, wobei bevorzugt wenigstens zwei Hohlräume mit unterschiedlichen Füllmaterialien befüllt werden. Insbesondere bei wenigstens zwei Hohlräumen können die beiden verschiedenen Füllmaterialien, die bevorzugt bereits während des additiven Fertigungsverfahrens in die Hohlräume eingefüllt werden, aus unterschiedlichen Komponenten beispielsweise eines Silikons bestehen, die miteinander chemisch reagieren. Derartige a-b-Silikone sind aus dem Stand der Technik bekannt und führen zu einem Aushärten oder Vernetzen oder Stabilisieren des Materials, sobald die beiden Komponenten miteinander in Kontakt kommen. Sie können verwendet werden, um beispielsweise einen Hohlraum, der beispielsweise zwischen einem Amputationsstumpf und einem Prothesenliner entsteht, wenn ein Standardgrößen-Liner verwendet wird, auszufüllen und so die Innenkontur des Liners individuell an die Körperform des Trägers der orthopädietechnischen Einrichtung anzupassen. Das vermischte aber noch flüssige Material umfließt dabei gekapselt in einer bereits ausgehärteten Hülle als flüssige Zwischenschicht den Stumpf, ohne mit diesem direkt in Kontakt zu kommen. Es nimmt die jeweilige Stumpfgeometrie an und härtet dann in dieser Form aus.

Eine Vorrichtung zum Durchführen eines derartigen Verfahrens verfügt folglich über wenigstens drei Ausgabedüsen, durch die die unterschiedlichen Materialien ausgegeben werden können. Während aus der einen Ausgabedüse das Fertigungsmaterial in die gewünschte Form beispielsweise in die Gelmatrix eingebracht wird, werden die unterschiedlichen Füllmaterialien aus den anderen Ausgabedüsen in die so entstehenden Hohlräume eingeführt. Sollen die beiden Füllmaterialien später miteinander zur Reaktion gebracht werden, muss lediglich eine Verbindungswand, die sich zwischen den beiden Hohlräumen befindet, getrennt werden.

Alternativ oder zusätzlich dazu werden die Hohlräume erst nach dem Aushärten des Fertigungsmaterials gefüllt. Beispielsweise sind aus der nicht vorveröffentlichten DE 10 2018 111 442 orthopädietechnische Einrichtungen, insbesondere Prothesenliner, bekannt, die Hohlräume aufweisen, die nach dem Aushärten des jeweiligen Materials, aus dem die Wände der Hohlräume bestehen, mit einem Füllmaterial befüllt werden. Dadurch kann die mechanische Stabilität an dieser Stelle erhöht werden, sodass beispielsweise eine Stützvorrichtung oder eine Stützstruktur innerhalb des Grundkörpers der orthopädietechnischen Einrichtung erzeugt werden kann. Durch eine entsprechende Ausgestaltung der hier beschriebenen Verfahren können auch mehrere Bauteile der orthopädietechnischen Einrichtung durch das additive Fertigungsverfahren aus dem wenigstens einen Fertigungsmaterial hergestellt werden, die jeweils über einen Hohlraum, beispielsweise in Form eines Schlauches oder Tunnels, verfügen und somit einander in Verbindung gebracht werden können, so dass diese unterschiedlichen Hohlräume miteinander in Kontakt kommen und insbesondere fluidtechnisch miteinander verbunden sind. Auf diese Weise können die Hohlräume gemeinsam mit dem jeweiligen strukturgebenden Material gefüllt werden. Selbstverständlich können auch andere Materialien, beispielsweise Kühlmaterialien oder wärmeabgebende Materialien, eingeführt werden, um die orthopädietechnische Einrichtung zu erwärmen oder zu kühlen und auf diese Weise den Tragekomfort für den Träger der orthopädietechnischen Einrichtung zu erhöhen.

In einer bevorzugten Ausgestaltung ist das Fertigungsmaterial, das in dem additiven Fertigungsverfahren verwendet wird, ein elastisches Material, wenn es fertig ausgehärtet ist. Insbesondere in diesem Fall, aber auch bei anderen Fertigungsmaterialien ist es von Vorteil, einen Hohlraum, der während des additiven Fertigungsverfahrens hergestellt wird, auszuschäumen. Dazu wird ein schäumbares Material in den fertig gestellten Hohlraum eingebracht, das anschließend aufschäumt und den Hohlraum vorzugsweise vollständig erfüllt. Dazu ist prinzipiell jedes aus dem Stand der Technik bekannte schäumbare Material verwendbar, wobei insbesondere mehrkomponentige Materialien verwendet werden. Dabei werden mindestens zwei Komponenten des zu schäumenden Materials in den Hohlraum eingebracht, die anschließend miteinander reagieren und zu schäumen beginnen.

Handelt es sich bei der orthopädietechnischen Einrichtung beispielsweise um einen Vakuumliner, also ein Prothesenliner, bei dem der Prothesenschaft durch Unterdruck, also durch Evakuieren des Zwischenraums zwischen dem Prothesenliner und dem Prothesenschaft, an dem Prothesenliner gehalten wird, ist es von Vorteil, in die orthopädietechnische Einrichtung, die in diesem Fall der Liner und/oder der Prothesenschaft sein kann, Hohlräume im Sinne von Evakuierungskanälen einzuarbeiten, um auf diese Weise den Unterdruck möglichst homogen in dem Zwischenraum zwischen Prothesenschaft und Prothesenliner hervorrufen zu können.

Vorteilhafterweise wird mittels des additiven Fertigungsverfahrens aus dem wenigstens einen Fertigungsmaterial wenigstens ein pneumatisches Element und/oder wenigstens ein hydraulisches Element hergestellt, vorzugsweise wenigstens ein Volumenreservoir, wenigstens eine Dichtlippe, wenigstens ein Ventil und/oder wenigstens eine Pumpe, wobei das hydraulische und/oder pneumatische Element bevorzugt einstückig mit einem anderen Bauteil der orthopädietechnischen Einrichtung hergestellt wird. Derartige Bauteile werden bei einer Reihe von orthopädietechnischen Einrichtungen verwendet und können auf diese Weise einfach und kostengünstig sowie optimal positioniert gefertigt werden. Eine Dichtlippe, die beispielsweise bei Vakuumlinern verwendet wird, um den zu evakuierenden Zwischenraum zwischen Prothesenliner und Prothesenschaft luftdicht nach außen abzudichten, wird in der Regel auf der Außenseite eines Prothesenliners angeordnet. Es können auch mehrere Dichtlippen an der Außenseite und/oder Innenseite des Prothesenliners angeordnet werden. Dabei kann mit dem hier beschriebenen Verfahren der gesamte Liner in einem additiven Fertigungsverfahren hergestellt werden, wobei dann das wenigstens eine Fertigungsmaterial das Material des Liners ist. Dabei kann es sich beispielsweise um ein Silikon oder ein Polyurethan handeln. In diesem Fall kann die Dichtlippe gleichzeitig mit dem übrigen Liner während des additiven Fertigungsverfahrens hergestellt werden, sodass eine optimale Haftung zwischen der Dichtlippe und dem Grundkörper des Liners, die dann durch eine stoffschlüssige Verbindung hervorgerufen wird, erreicht wird. Alternativ dazu kann die Dichtlippe auch nachträglich an einem bereits fertig und separat gefertigten Grundkörper eines Liners angeordnet werden. Dies ist insbesondere dann von Vorteil, wenn zwar ein Standardliner für die Patientenversorgung verwendet wird, jedoch die optimale Position der Dichtlippe an der Außenseite des Liners beispielsweise von der Länge des Amputationsstumpfes abhängt und damit individuell für den Träger der orthopädietechnischen Einrichtung angepasst werden muss. Die individuelle Anpassung kann durch das additive Fertigungsverfahren erfolgen.

In einer Reihe von Prothesenlinern wird innerhalb des Liners beispielsweise eine Luftleitung mit einem Flatterventil oder mit einem Einwegeventil verwendet, um die Hubbewegung zwischen dem Prothesenliner und dem Prothesenschaft, die beim Gehen auftritt, zum Abpumpen und zum Evakuieren des Volumens zu verwenden. Diese pneumatischen Elemente können mit dem hier beschriebenen Verfahren in den Grundkörper des Liners, der aus dem wenigstens einen Fertigungsmaterial herstellbar ist, eingearbeitet werden. Die US 2017/0143519 A1 betrifft einen Prothesenliner mit einem Ein-Wege-Ventil und einem eingegossenen, luftdurchlässigen Material, das einen Strömungsweg bildet. Eine Pumpkammer kann zwischen einem Außenliner und einem Innenliner eingesetzt sein.

Ähnlich einer Dichtlippe können beispielsweise auch Verriegelungselemente für die mechanische Verrieglung zweier Bauteile der orthopädietechnischen Einrichtung miteinander durch das additive Fertigungsverfahren, insbesondere durch das Rapid Liquid Printing-Verfahren, an ein separat oder zeitgleich angefertigtes Bauteil, beispielsweise den Grundkörper eines Liners, angeformt werden. Das Rapid-Liquid-Printing-Verfahren ist beispielsweise in der US 2018-281295 A1 beschrieben.

In einer bevorzugten Ausgestaltung werden bei dem Verfahren zunächst Messdaten von einem Patienten erfasst, die dann einer elektrischen und/oder elektronischen Steuerung zur Verfügung gestellt werden. Diese Steuerung ist eingerichtet, zumindest auch auf der Grundlage dieser Messdaten das additive Fertigungsverfahren zu steuern. Dies ist insbesondere für orthopädietechnische Einrichtungen von Vorteil, die individuell an den Patienten angepasst werden. Insbesondere Prothesenschäfte und Prothesenliner können auf diese Weise schnell, einfach, kostengünstig und dennoch individuell für jeden Patienten einzeln gefertigt werden. Zur Erfassung der Messdaten sind aus dem Stand der Technik unterschiedliche Verfahren bekannt. So kann beispielsweise der Körperteil des Patienten, der mit der orthopädietechnischen Einrichtung in Kontakt kommen wird, von einem optischen Scanner erfasst und dreidimensional vermessen werden. Die so ermittelten Messdaten werden der elektrischen und/oder elektronischen Steuerung zur Verfügung gestellt. Der Amputationsstumpf oder das Körperteil, das vermessen werden soll, kann dabei in eine spezielle Vorrichtung eingeführt, eingehängt oder eingelegt werden, so dass die Druckverhältnisse, die bei angelegter Prothese oder Orthese herrschen sollen und die selbstverständlich die geometrische Form des Körperteils und des Amputationsstumpfes beeinflussen, in Betracht gezogen werden können.

Neben der beschriebenen Herstellung individuell angepasster orthopädietechnischer Einrichtungen lassen sich durch die hier beschriebenen Ausgestaltungen des Verfahrens auch standardisierte Formen und/oder Größen der orthopädietechnischen Einrichtungen einfacher und kostengünstiger als im Stand der Technik herstellen, da keine Gießformen hergestellt werden müssen. Das Fertigungsmaterial wird einfach innerhalb des Stützmaterials in der gewünschten Anordnung und Verteilung positioniert und härtet dann aus oder wird ausgehärtet.

Zudem können semi-individuelle Ausgestaltungen orthopädietechnischer Einrichtungen hergestellt werden, bei denen beispielsweise digital hinterlegte Standardformen mittels einzelner von dem Patienten genommener Maße individualisiert werden. Diese können dann mittels des additiven Fertigungsverfahrens einfach und schnell hergestellt werden. So kann beispielsweise eine Position und/oder Größe einer Dichtlippe an der Außenseite eines Prothesenliners patientenindividuell angepasst werden. Auch Prothesenhandschuhe können beispielsweise hinsichtlich ihrer Länge und/oder Breite individuell angefertigt werden, während ansonsten Standardmaße verwendet werden.

In bevorzugten Ausgestaltungen des hier beschriebenen Verfahrens kann beispielsweise die Haptik oder die Struktur der Oberfläche der orthopädietechnischen Einrichtung oder des Bauteils der orthopädietechnischen Einrichtung, die oder das durch das additive Fertigungsverfahren hergestellt wird, beeinflusst und gegebenenfalls bereichsweise geändert werden. So kann beispielsweise ein Prothesenliner hergestellt werden, der im proximalen Bereich eine andere Haptik oder Struktur aufweist als im distalen Bereich. Auf diese Weise kann beispielsweise das Anziehen vereinfacht werden. Auch die Hafteigenschaften können variiert werden, indem beispielsweise an der Innenseite eines Prothesenliners, der durch das additive Fertigungsverfahren hergestellt wird, eine Oberflächenstruktur zumindest bereichsweise, gegebenenfalls auch vollständig, aufgebracht wird. Durch die bereits angesprochenen Evakuierungskanäle für eine Unterdruckversorgung eines Prothesenliners kann auch die Unterdruckverteilung bei einem Unterdruckliner individuell angepasst und für den Tragekomfort des Patienten optimal gewählt werden. Dazu werden in die orthopädietechnische Einrichtung Evakuierungskanäle eingebracht, was vorzugsweise während des additiven Fertigungsverfahrens geschieht. Durch diese Evakuierungskanäle kann das Volumen evakuiert werden, wobei durch Form und Gestaltung der Kanäle die Unterdruckverteilung individualisiert ist.

Insbesondere durch die Verwendung unterschiedlicher Fertigungsmaterialien lässt sich zudem beispielsweise ein Shore-Härtegrad des jeweiligen ausgehärteten Fertigungsmaterials wählen. So ist es beispielsweise auch möglich, eine verschleißbeständige Außenschicht und ein weiches, polsterndes Kernmaterial gleichzeitig herzustellen, indem zwei unterschiedliche Fertigungsmaterialien verwendet werden. Zudem lässt sich das Fertigungsmaterial in einer schaumartigen Struktur anordnen, durch die eine atmungsaktive Wandung erzeugt werden kann.

Vorzugsweise ist die orthopädietechnische Einrichtung ein Prothesenliner zum Einsatz in einem Prothesenschaft, wobei der Prothesenschaft einen Aufnahmeraum mit einem distalen Ende und einem proximalen Rand aufweist. Der Aufnahmeraum ist so ausgebildet, dass der mit dem Prothesenliner versorgte Amputationsstumpf in ihn eingeführt und in ihm aufgenommen werden kann. Das Verfahren beinhaltet das Festlegen eines Dichtlippenverlaufes an einer Außenseite des Prothesenliners korrespondierend zu dem Verlauf einer Höhenkontur des Prothesenschaftes oder anhand vorhandener, bekannter anatomischer Gegebenheiten eines Amputationsstumpfes und das Anordnen einer Dichtlippe an der Außenseite des Prothesenliners entlang des festgelegten Dichtlippenverlaufes mittels des wenigstens einen additiven Fertigungsverfahrens. Dies beinhaltet auch das Fertigen eines Liners oder eines Linergrundkörpers, an dem die Dichtlippe einstückig angeformt ist.

Die Höhenkontur ist der Verlauf des proximalen Randes des Prothesenschaftes in Proximal-Distal-Richtung und bildet den Verlauf der proximalen Abschlussgeometrie des Prothesenschaftes. Nach der Bestimmung der Höhenkontur wird ein Dichtlippenverlauf an der Außenseite des Prothesenliners korrespondierend zu dem Verlauf der Höhenkontur des Prothesenschaftes festgelegt. Entlang des festgelegten Dichtlippenverlaufes wird eine Dichtlippe an der Außenseite des Prothesenliners angeordnet. Dies geschieht mittels des wenigstens einen additiven Fertigungsverfahrens.

Der Dichtlippenverlauf folgt somit im angelegten Zustand der Höhenkontur des proximalen Randes des Prothesenschaftes und ist korrespondierend zu der oberen Abschlussgeometrie des Prothesenschaftes ausgebildet. Die Position der Dichtlippe an dem Prothesenliner ist dabei so festgelegt, dass die Dichtlippe distal zu dem proximalen Rand des Prothesenschaftes angeordnet ist, wenn der an den Amputationsstumpf angelegte Prothesenliner vollständig in den Prothesenschaft eingeführt ist. Durch den korrespondierenden Verlauf der Dichtlippe zu der proximalen Abschlussgeometrie des Prothesenschaftes ist es möglich, die Dichtlippe so anzuordnen, dass sie im angezogenen Zustand des Prothesenliners und des Prothesenschaftes möglichst weit proximal verläuft, wodurch das zu evakuierende Volumen zwischen dem Prothesenliner und dem Prothesenschaft gegenüber den Ausgestaltungen aus dem Stand der Technik vergrößert wird. Dadurch wird die Kraft, mit der der Prothesenschaft an dem Prothesenliner gehalten wird, vergrößert, ohne dass der Druck innerhalb des evakuierten Volumens gegenüber den Ausgestaltungen aus dem Stand der Technik reduziert werden muss. Gleichzeitig kommt es zu einer möglichst großflächigen und damit gleichmäßigen Kraftverteilung der zum Halten des Prothesenschaftes an dem Prothesenliner nötigen Kraft am Amputationsstumpf.

Durch die individuell an den proximalen Rand des Prothesenschaftes angepasste Geometrie und einen entsprechenden Verlauf der Dichtlippe wird die mechanische Güte des Interfaces maximiert und die Belastungen auf den Stumpf, insbesondere den Amputationsstumpf, reduziert. Dies erhöht den Komfort für den Anwender und stellt eine sichere Verbindung zwischen dem Prothesenschaft und dem Stumpf her.

Der Dichtlippenverlauf kann z.B. anhand eines vorhandenen oder errechneten, insbesondere in digitaler Form vorliegenden Verlaufes des proximalen Randes eines Prothesenschaftes festgelegt werden. Der bekannte Verlauf des proximalen Randes des Prothesenschaftes dient als Referenz für den Dichtlippenverlauf, der entsprechend festgelegt wird. Ebenso kann die Festlegung des Dichtlippenverlaufes aufgrund eines gescannten Stumpfes oder anderweitig erhaltener Daten über die Form und/oder Beschaffenheit des Stumpfes oder dessen anatomischer Gegebenheiten erfolgen. Die digital vorliegende oder errechnete Anatomie kann als Grundlage für den zu fertigenden Prothesenliner und zudem für den zu fertigenden Prothesenschaft dienen. Der Prothesenliner und ggf. auch der Prothesenschaft werden digital um das Modell oder digitale Abbild des Stumpfes herum modelliert. Die Form korrespondiert im Wesentlichen zu der Außenkontur des Stumpfes, mit Zuschlägen für Polster an dem Liner und ggf. Anpassungen für den Prothesenschaft für Entlastungsbereiche oder Kompressionsbereiche zum Ausgleichen von Volumenschwankungen. Der Prothesenliner kann ebenfalls nur anhand des Stumpfmodells oder des digitalen Abbildes des Stumpfes und/oder des Prothesenschaftes entworfen und in einen digitalen Datensatz umgewandelt werden, wobei der Dichtlippenverlauf anhand der anatomischen Gegebenheiten ohne bereits vorhandenen oder errechneten Prothesenschaftdatensatz bestimmt werden kann.

In einer Weiterbildung der Erfindung wird vor dem Festlegen des Dichtlippenverlaufes die Höhenkontur des proximalen Randes des Prothesenschaftes ermittelt. Die Höhenkontur, also der Verlauf in Proximal-Distal-Richtung des proximalen Randes des Prothesenschaftes, bildet den Verlauf der oberen Abschlussgeometrie des Prothesenschaftes. Neben der Erfassung einer Höhenkontur eines bereits physisch vorhandenen Prothesenschaftes, beispielsweise durch ein Abtastverfahren, Entlangfahren eines Höhensensors auf dem proximalen Rand oder parallel dazu und Zuordnen der Höhendaten zu den Umfangskoordinaten oder durch eine berührungslose Messung, beispielsweise optische Messverfahren oder anderweitiges Scannen, kann die Festlegung auch auf der Grundlage allein von Daten über einen Prothesenschaft, insbesondere eines noch zu fertigenden Prothesenschaftes, erfolgen. Existiert bereits ein 3D-Modell oder ein Datensatz, anhand dessen der Prothesenschaft gefertigt werden soll, kann der Dichtlippenverlauf auf der Grundlage dieser Daten festgelegt und eine Fertigung des Prothesenliners erfolgen.

Die Dichtlippe ist vorzugsweise in distaler Richtung zu dem proximalen Rand des Prothesenschaftes versetzt an dem Prothesenliner angeordnet, um zu vermeiden, dass im angezogenen, vollständig eingeführten Zustand des Prothesenliners die Dichtlippe proximal über den proximalen Rand des Prothesenschaftes hinaussteht. Beim Erfassen der Höhenkontur wird vorzugsweise nicht nur der Verlauf des proximalen Randes des Prothesenschaftes in Proximal-Distal-Richtung, sondern auch der Abstand der Höhenkontur von dem distalen Ende des Aufnahmeraumes ermittelt. Aus dem so ermittelten Abstand kann bestimmt werden, in welchem Abstand von einem distalen Ende des Prothesenliners der proximale Rand des Prothesenschaftes an dem Prothesenliner anliegen wird, wenn der Prothesenliner an einem Amputationsstumpf in den Prothesenschaft eingeführt worden ist. Von dieser Linie ausgehend wird die Dichtlippe vorzugsweise nach distal versetzt an dem Prothesenliner angeordnet. Durch die Verschiebung oder Verlagerung in distaler Richtung von dem proximalen Rand des Prothesenschaftes weg wird eine Sicherheitszone eingerichtet, durch die beispielsweise Abweichungen in der vorgesehenen Orientierung des Prothesenliners an dem Stumpf ausgeglichen werden können.

Das Erfassen des Verlaufs der Höhenkontur erfolgt in Proximal-Distal-Richtung und berücksichtigt vorzugsweise die Gesamtlänge des Prothesenschaftes und damit auch die Entfernung des Dichtlippenverlaufes von dem distalen Ende des Prothesenliners. Alternativ oder ergänzend wird vorzugsweise eine Umfangskontur des proximalen Randes des Prothesenschaftes über den Umfang erfasst. Damit ist es möglich, eine Erstreckung der Dichtlippe in radiale Richtung, also einen Abstand der radial außen liegenden Kante der Dichtlippe zu der Außenseite des Prothesenliners, an den jeweiligen Patienten anzupassen. Die Erstreckung der Dichtlippe ist vorzugsweise über den Umfang nicht konstant, sondern variiert je nach erwartetem Abstand des Amputationsstumpfes und der Außenseite des darüber gezogenen Liners von der Innenseite des Prothesenschaftes.

Die Dichtlippe ist vorzugsweise über den Umfang des Prothesenliners in Proximal-Distal-Richtung äquidistant zu dem proximalen Rand des Prothesenschaftes angeordnet werden, verläuft also zumindest im Wesentlichen, vorzugsweise jedoch vollständig identisch zu der Höhenkontur des proximalen Randes des Prothesenschaftes verlaufen.

Die Dichtlippe wird vorzugsweise in einem Dichtlippenbereich angeordnet, der in Proximal-Distal-Richtung breiter als die Dichtlippe selbst ist und einen Montagebereich darstellt, innerhalb dessen die Dichtlippe an der Außenseite eines bereits vorgefertigten Prothesenliners angeordnet und festgelegt werden kann. Der Dichtlippenbereich dient zur Erleichterung der Fertigung und ermöglicht es in dem wenigstens einen additiven Fertigungsverfahren die Dichtlippe selbst innerhalb eines vorgegebenen Bereiches in Proximal-Distal-Richtung an der Außenseite des Prothesenliners anzuordnen. Die proximale und distale Grenze des Dichtlippenbereiches ist vorzugsweise abhängig von und korrespondiert besonders bevorzugt zu der Höhenkontur des proximalen Randes des Prothesenschaftes.

Der Dichtlippenbereich ist vorzugsweise doppelt so breit wie die Dichtlippe an ihrem Übergang zu der Außenseite des Prothesenliners, also der an der Außenseite des Prothesenliners angeordneten Basis der Dichtlippe.

Die Höhenkontur oder die Höhenkontur und die Umfangskontur des Prothesenschaftes werden bevorzugt optisch erfasst. Die erfassten Bilddaten bilden beispielsweise eine Basis für ein digitales 3D-Modell, für das oder von dem ein Datensatz erstellt wird. Aufgrund des Datensatzes des 3D-Modells werden der Dichtlippenverlauf oder der Dichtlippenverlauf und die Dichtlippenform in Abhängigkeit von der erfassten Höhenkontur oder der erfassten Umfangs- und Höhenkontur festgelegt. Die Erfassung der Höhenkontur kann auch aus bereits vorliegenden Daten, etwa einem 3D-Modell des Schaftes, erfolgen, ohne dass ein Prothesenschaft physisch vorhanden sein muss. Wird beispielsweise ein Prothesenschaft in einem additiven oder in einem anderen Fertigungsverfahren auf der Grundlage eines Datensatzes eines Gliedmaßenstumpfes, beispielsweise eines Amputationsstumpfes, erstellt, folgt die Innenkontur des Prothesenschaftes im Wesentlichen der Außenkontur des Stumpfes mit einer Zugabe für Volumenschwankungen und gegebenenfalls die Materialstärke des Prothesenliners.

Wird der Prothesenschaft nicht auf der Basis von digitalen Daten, die beispielsweise von dem Stumpf selbst oder einem Gipsmodell davon abgenommen werden, hergestellt, wird vorzugsweise die Innenkontur eines bereits vorhandenen Prothesenschaftes optisch oder auf andere Art und Weise erfasst und in einem Computersystem gespeichert werden. Anhand des Vergleiches der Außenkontur des Stumpfes und der Innenkontur des Prothesenschaftes und der ebenfalls im Rahmen des 3D-Modells vorhandenen Höhenkontur des proximalen Randes des Prothesenschaftes wird dann der Dichtlippenverlauf an der Außenseite des Prothesenliners festgelegt. Dies beinhaltet selbstverständlich auch die Position der Dichtlippe an der Außenseite des Prothesenliners. Zusätzlich wird vorzugsweise die Höhe und die Form sowie die Dicke des Prothesenliners bzw. eines Grundkörpers des Prothesenliners festgelegt und als Datensatz zur Fertigung in dem wenigstens einen additiven Fertigungsverfahren verarbeitet werden. Im Rahmen des wenigstens einen additiven Fertigungsverfahrens, beispielsweise im Rahmen eines Rapid-Liquid-Printing-Verfahrens wird der Prothesenliner mit dem an die Höhenkontur und/oder Umfangskontur angepassten Dichtlippenverlauf und Dichtlippenhöhe und/oder Dichtlippendicke hergestellt werden.

Der Verlauf der Dichtlippe kann auch direkt aus anatomischen Daten bestimmt werden, etwa auf Grundlage eines Scans eines Stumpfes. Die Höhenkontur und die Umfangskontur des Schaftes können dann entweder aus dem zunächst als geeignet oder optimal erachteten Verlauf der Dichtlippe bestimmt werden, so dass der Dichtlippenverlauf als Bezugsgröße für den Verlauf des proximalen Randes des Prothesenschaftes dient. Oder die Höhenkontur und die Umfangskontur des Schaftes werden ebenfalls aus den anatomischen Daten des Scans ermittelt. Im Ergebnis korrespondiert der Verlauf der Dichtlippe zu dem Verlauf der Höhenkontur des Prothesenschaftes, unabhängig davon ob der Dichtlippenverlauf in Abhängigkeit der zuerst festgelegten Höhenkontur des Prothesenschaftes, die Höhenkontur des Prothesenschaftes in Abhängigkeit von dem zunächst bestimmten Dichtlippenverlauf oder der Dichtlippenverlauf und die Höhenkontur unabhängig voneinander auf der Grundlage der anatomischen Gegebenheiten anhand z.B. des digitalen 3D-Stumpfmodells erstellt und festgelegt werden.

Die Dichtlippenhöhe kann in Abhängigkeit von einem erfassten Abstand zwischen einer Innenseite des Prothesenschaftes und der Außenseite des einzuführenden Stumpfes, über den gegebenenfalls der Grundköper des Prothesenliners gezogen wurde, festgelegt werden.

Mit einem auf diese Weise hergestellten Prothesenliner wird bevorzugt ein System aus einem Prothesenschaft und dem Prothesenliner gebildet, bei dem der Prothesenschaft einen Aufnahmeraum für einen mit dem Prothesenliner versorgten Stumpf aufweist. Der Prothesenschaft weist ein distales Ende und einen proximalen Rand auf. Der proximale Rand des Prothesenschaftes weist eine Höhenkontur und eine Umfangskontur auf. An der dem Prothesenschaft zugewandten Außenseite des Prothesenliners ist zumindest eine Dichtlippe ausgebildet oder festgelegt, wobei der Dichtlippenverlauf der zumindest einen Dichtlippe in dem vollständig eingeführten Zustand des Prothesenliners mit dem Verlauf der Höhenkontur des Prothesenschaftes korrespondiert. Die Dichtlippe muss nicht bündig mit dem proximalen Rand des Prothesenschaftes abschließen, vielmehr ist vorgesehen, dass die Dichtlippe der Abschlusskontur folgend distal vom proximalen Rand des Prothesenschaftes versetzt an dem Prothesenliner angeordnet ist.

Der Prothesenschaft ist distal zu der zumindest einen Dichtlippe im vollständig eingeführten Zustand des angelegten Prothesenliners geschlossenwandig ausgebildet, um einen möglichst großflächigen Interfacebereich herzustellen, sodass auf einer möglichst großen Fläche ein Unterdruck erzeugt werden kann, durch den die benötigte Haltekraft erzeugt und auf den Amputationsstumpf übertragen wird.

Der Prothesenschaft ist bevorzugt formstabil ausgebildet, um eine ausreichende Stabilität für die Aufnahme des Stumpfes mit dem Liner und die Anordnung weiterer Prothesenkomponenten, beispielsweise Prothesengelenke, bereitzustellen. Die zumindest eine Dichtlippe ist an einem Grundkörper des Prothesenliners befestigt oder ausgebildet und kann über den Umfang des Prothesenliners eine ungleichmäßige Höhe aufweisen, also radial nach außen unterschiedlich weit von der Außenseite des Prothesenliners abstehen, um Formschwankungen oder Formdifferenzen zwischen der Außenkontur des Stumpfes und der Innenkontur des Prothesenschaftes auszugleichen.

Eine Weiterbildung der Erfindung sieht vor, dass die zumindest eine Dichtlippe an einem Grundkörper des Prothesenliners befestigt oder ausgebildet ist und eine über den Umfang des Prothesenliners ungleichmäßige Höhe, also eine ungleichmäßige Erstreckung nach radial außen von der Außenseite des Prothesenliners, aufweist. Dadurch können Unterschiede in dem radialen Abstand zwischen der Außenseite des Grundkörpers im angelegten Zustand und der Innenseite des Prothesenschaftes ausgeglichen werden. Dadurch wird sichergestellt, dass die Dichtlippe immer an der Innenwand des Prothesenschaftes anliegt, wenn der Stumpf mit dem Liner eingeführt wird. Die über den Umfang des Prothesenliners unterschiedliche Höhe, also die unterschiedliche radiale Ausdehnung wird beispielsweise bei einem Vergleich zwischen der gescannten Innenseite des Prothesenschaftes und der gescannten Außenseite oder dem 3D-Modell des Stumpfes ermittelt.

Der Dichtlippenverlauf liegt bevorzugt nicht in einer Ebene und ist somit nicht geradlinig, sondern beschreibt eine Raumkurve mit einem über dem Umfang unregelmäßigen Abstand zu dem distalen Ende des Prothesenliners oder des Prothesenschaftes.

Der Prothesenliner für ein oben beschriebenes System weist zumindest eine an der Außenseite des Prothesenliners ausgebildete oder festgelegte Dichtlippe auf, die einen Dichtlippenverlauf in Gestalt einer Raumkurve ausbildet. Der Dichtlippenverlauf der zumindest einen Dichtlippe ist zu dem Verlauf einer Höhenkontur eines proximalen Randes eines Prothesenschaftes, in den der an einen Stumpf angelegte Prothesenliner eingeführt werden soll, korrespondierend ausgebildet ist.

Die Erfindung löst die gestellt Aufgabe zudem durch eine orthopädietechnische Einrichtung, insbesondere ein Prothesenliner, die nach einem der hier beschriebenen Verfahren hergestellt oder herstellbar ist.

Die vorliegende Anmeldung offenbart folgende Erfindung:
1. Verfahren zum Herstellen einer orthopädietechnischen Einrichtung, insbesondere eines Prothesenliners (2), **dadurch gekennzeichnet, dass** die orthopädietechnische Einrichtung zumindest teilweise mittels eines additiven Fertigungsverfahrens aus wenigstens einem Fertigungsmaterial (12) hergestellt wird, das in einem fließfähigen Zustand in ein Stützmaterial eingebracht wird und danach aushärtet.
   Die Anmeldung offenbart folgende Ausführungsbeispiele:
2. Verfahren nach Punkt 1, dadurch gekennzeichnet, dass Fertigungsmaterial (12) während des Aushärtens von dem Stützmaterial gestützt und/oder in seiner Position in einem Arbeitsraum gehalten wird und vorzugsweise ein selbsthärtendes oder durch Temperaturerhöhung härtbares Material ist.
3. Verfahren nach Punkt 1 oder 2, dadurch gekennzeichnet, dass das Fertigungsmaterial (12) aus wenigstens zwei Komponenten zusammengesetzt wird, wobei vorzugsweise ein Mischungsverhältnis der wenigstens zwei Komponenten während des additiven Fertigungsverfahrens einstellbar ist.
4. Verfahren nach Punkt 3, dadurch gekennzeichnet, dass das Fertigungsmaterial (12) nach dem Aushärten eine Shore-Härte aufweist, deren Wert von dem Mischungsverhältnis abhängt.
5. Verfahren nach einem der vorstehenden Punkte, dadurch gekennzeichnet, dass in dem additiven Fertigungsverfahren wenigstens zwei unterschiedliche Fertigungsmaterialien (12), vorzugsweise gleichzeitig, verwendet werden.
6. Verfahren nach einem der vorstehenden Punkte, dadurch gekennzeichnet, dass das wenigstens eine Fertigungsmaterial (12) während des additiven Fertigungsverfahrens mit einem separat gefertigtes Bauteil der orthopädietechnischen Einrichtung verbunden wird.
7. Verfahren nach einem der vorstehenden Punkte, dadurch gekennzeichnet, dass in dem additiven Fertigungsverfahren die Wandstärke der orthopädietechnischen Einrichtung kontinuierlich oder in diskreten Schritten variiert wird, sodass mindestens eine Ausbuchtung, Vertiefung, Aufdickung, Verjüngung und/oder ein Hinterschnitt erzeugt wird.
8. Verfahren nach einem der vorstehenden Punkte, dadurch gekennzeichnet, dass mit dem additiven Fertigungsverfahren eine orthopädietechnische Einrichtung mit wenigstens einem Hohlraum erzeugt wird.
9. Verfahren nach Punkt 8, dadurch gekennzeichnet, dass der wenigstens eine Hohlraum während des additiven Fertigungsverfahrens mit wenigsten einem Füllmaterial zumindest teilweise, bevorzugt vollständig, gefüllt wird, wobei bevorzugt wenigstens zwei Hohlräume mit unterschiedlichen Füllmaterialien befüllt werden.
10. Verfahren nach einem der vorstehenden Punkte, dadurch gekennzeichnet, dass mittels des additiven Fertigungsverfahrens aus dem wenigstens einen Fertigungsmaterial (12) wenigstens ein pneumatisches und/oder wenigstens ein hydraulisches Element, vorzugsweise wenigstens ein Volumenreservoir (46), wenigstens eine Dichtlippe (16), wenigstens ein Ventil und/oder wenigstens eine Pumpe hergestellt wird, das bevorzugt einstückig mit einem anderen Bauteil der orthopädietechnischen Einrichtung hergestellt wird.
11. Verfahren nach einem der vorstehenden Punkte, dadurch gekennzeichnet, dass Messdaten von einem Patienten erfasst und einer elektrischen und/oder elektronischen Steuerung zur Verfügung gestellt werden, die eingerichtet ist, zumindest auch auf der Grundlage der Messdaten das additive Fertigungsverfahren zu steuern.
12. Verfahren nach einem der vorstehenden Punkte, dadurch gekennzeichnet, dass die orthopädietechnische Einrichtung ein Prothesenliner (2) zum Einsatz in einem Prothesenschaft (80) ist, wobei der Prothesenschaft (80) einen Aufnahmeraum (84) mit einem distalen Ende (88) und einem proximalen Rand (82) aufweist, wobei das Verfahren folgende Schritte aufweist:
   a) Festlegen eines Dichtlippenverlaufes an einer Außenseite (76) des Prothesenliners (2) korrespondierend zu dem Verlauf einer Höhenkontur des Prothesenschaftes (80) oder anhand vorhandener, bekannter anatomischer Gegebenheiten eines Amputationsstumpfes und
   b) Anordnen einer Dichtlippe (16) an der Außenseite (76) des Prothesenliners (2) entlang des festgelegten Dichtlippenverlaufes mittels des wenigstens einen additiven Fertigungsverfahrens.
13. Verfahren nach Punkt 12, dadurch gekennzeichnet, dass die Dichtlippe (16) in distaler Richtung zu dem proximalen Rand (82) des Prothesenschaftes (80) versetzt, insbesondere äquidistant an dem Prothesenliner (2) angeordnet ist.
14. Verfahren nach Punkt 12 oder 13, dadurch gekennzeichnet, dass Höhenkontur des Prothesenschaftes (80) optisch erfasst wird, ein digitales 3D-Modell erstellt wird und der Dichtlippenverlauf in Abhängigkeit von der erfassten Höhenkontur festgelegt wird.
15. Orthopädietechnische Einrichtung, hergestellt oder herstellbar nach einem Verfahren gemäß einem der vorstehenden Punkte.

Mit Hilfe der beiliegenden Zeichnungen werden nachfolgend einige Ausführungsbeispiele der vorliegenden Erfindung näher erläutert.

Es zeigt:
- Figur 1 -: die schematische Darstellung eines Prothesenliners mit unterschiedlichen Komponenten,
- Figur 2 -: die schematische Darstellung eines Prothesenliners mit unterschiedlichen Designelementen,
- Figur 3 -: die schematische Darstellung eines Prothesenliners mit unterschiedlichen Oberflächenstrukturierungen,
- Figur 4 -: die schematische Darstellung eines Prothesenliners mit unterschiedlichen Materialstrukturierungen,
- Figur 5 -: die schematische Teilübersicht über mit dem Verfahren gemäß einem Ausführungsbeispiel der vorliegenden Erfindung herzustellenden Komponenten,
- Figur 6 -: die schematische Darstellung unterschiedlicher Fertigungsverfahren auf Maß
- Figur 7 -: die schematische Darstellung eines Teilstandard- und eines Standardprothesenliners,
- Figur 8 -: einen Prothesenliner in Einzeldarstellung
- Figur 9 -: ein System aus Prothesenschaft und einem darin angeordneten Prothesenliner und
- Figur 10-: eine schematische Darstellung einer Variante des Herstellverfahrens.

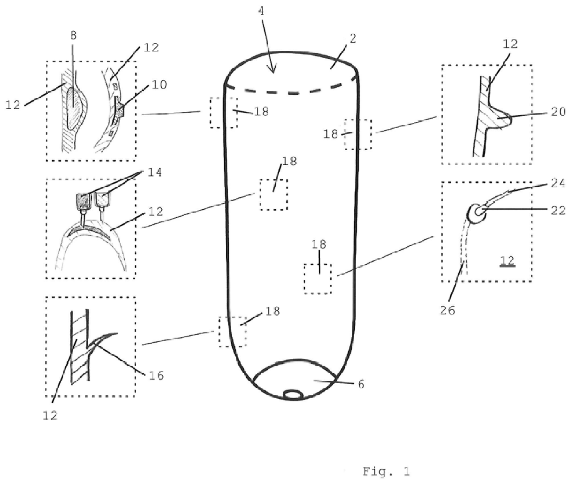

Figur 1 zeigt im zentralen Bereich einen Prothesenliner 2, der gemäß einem Ausführungsbeispiel der vorliegenden Erfindung hergestellt wurde. Der Prothesenliner 2 verfügt im proximalen Bereich über eine Öffnung 4 und im distalen Bereich über eine Linerkappe 6. In den kleinen Ausschnitten, die durch gestrichelte Linien begrenzt sind, sind unterschiedliche Komponenten dargestellt, die an dem Prothesenliner 2 angeordnet sein können.

Oben links sind separat gefertigte Bauteile in Form beispielsweise eines Kissens 8 und einer Elektrode 10 dargestellt, die im gezeigten Ausführungsbeispiel von dem Grundkörper des Prothesenliners 2 und dessen Fertigungsmaterial 12 umgeben sind. In dem darunter liegenden Kasten ist eine Ausgestaltung gezeigt, in der zwei unterschiedliche Fertigungsmaterialien über zwei Zuführungen 14 simultan verarbeitet werden. Es ist auf diese Weise beispielsweise möglich, ein Fertigungsmaterial höherer Härte als Versteifungselement in ein weicheres Fertigungsmaterial einzubringen, das beispielsweise ein Linermaterial für einen Grundkörper des Prothesenliners 2 ist.

Im untersten Kasten auf der linken Seite ist eine Dichtlippe 16 dargestellt, die beispielsweise als separat gefertigtes Bauteil ausgebildet sein kann, an das das Fertigungsmaterial 12 mit dem additiven Fertigungsverfahren angedruckt wird. Bevorzugt werden der Grundkörper des Liners 2 und die Dichtlippe 16 in einem einzigen Fertigungsschritt, nämlich dem additiven Fertigungsverfahren, zusammen hergestellt. Alternativ dazu kann auch der Grundkörper des Prothesenliners 2 als separat gefertigtes Bauteil vorliegen, an das die Dichtlippe 16 angedruckt wird. Die Positionskästchen 18 sollen lediglich beispielhaft darstellen, dass die gezeigten Komponenten an unterschiedlichsten Positionen des Prothesenliners 2 angeordnet werden können.

Auf der rechten Seite der Figur 1 ist im oberen Bereich dargestellt, dass das Fertigungsmaterial 12 in Form einer Wulst 20, die beispielsweise ein Verriegelungselement sein kann, vorliegt. Auch dies ist durch ein Verfahren gemäß einem Ausführungsbeispiel der vorliegenden Erfindung problemlos herstellbar. Die Wulst 20 kann dabei als separates Bauteil ausgebildet sein, an das während des additiven Fertigungsverfahrens das Fertigungsmaterial 12 angedruckt wird. Alternativ oder zusätzlich dazu kann eine Wulst 20 auch aus dem Fertigungsmaterial 12 oder einem zweiten Fertigungsmaterial im additiven Fertigungsverfahren hergestellt sein. Bevorzugt werden der Grundkörper des Liners 2 und die Wulst 20 in einem einzigen Fertigungsschritt, nämlich dem additiven Fertigungsverfahren, zusammen hergestellt.

Unten rechts in Figur 1 ist dargestellt, dass ein Anschlusselement 22 an das Fertigungsmaterial 12 angedruckt ist. Über ein derartiges Anschlusselement 22 kann beispielsweise ein Kabel 24 mit einem sich im Inneren des Fertigungsmaterials 12 befindlichen elektrischen Leiter 26 verbunden werden. Dieser kann beispielsweise mit einer Elektrode, die in Figur 1 nicht dargestellt ist, verbunden sein.

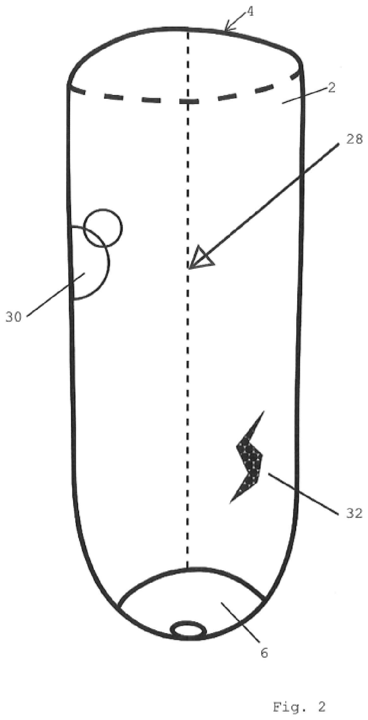

Figur 2 zeigt den Prothesenliner 2 mit unterschiedlichen optischen Elementen, die durch eingefärbte Fertigungsmaterialien 12 mit dem additiven Fertigungsverfahren hergestellt werden können. Von der Öffnung 4 bis zur distalen Linerkappe 6 erstreckt sich eine Markierungslinie 28, durch die es dem Träger der orthopädietechnischen Einrichtung erleichtert wird, die orthopädietechnische Einrichtung, im gezeigten Ausführungsbeispiel also den Prothesenliner 2, in der richtigen Orientierung anzulegen. Selbstverständlich ist es nicht notwendig, dass die Markierungslinie 28 von der proximalen Öffnung 4 bis zur distalen Linerkappe 6 verläuft.

Im linken Bereich des Prothesenliners 2 ist ein Designelement 30 dargestellt, das im Wesentlichen eine ästhetische Aufgabe erfüllt. So kann es beispielsweise als Logo ausgebildet sein, durch das die orthopädietechnische Einrichtung als vom Hersteller kommend gekennzeichnet werden kann.

Das dritte optische Element ist ein Verschleißindikator 32, der ebenfalls aus einem eingefärbten Fertigungsmaterial besteht. Ein solcher Verschleißindikator 32 kann beispielsweise realisiert werden, indem ein Grundkörper des Prothesenliners 2 mehrlagig ausgebildet ist. Dies bedeutet, dass im additiven Fertigungsverfahren mehrere Fertigungsmaterialien verwendet werden, die sich zumindest auch in der Farbe unterscheiden. Ist die äußere Lage des Prothesenliners 2 defekt oder verschlissen, ist die andere Farbe der jeweiligen unteren Lage erkennbar und wirkt als Verschleißindikator 32.

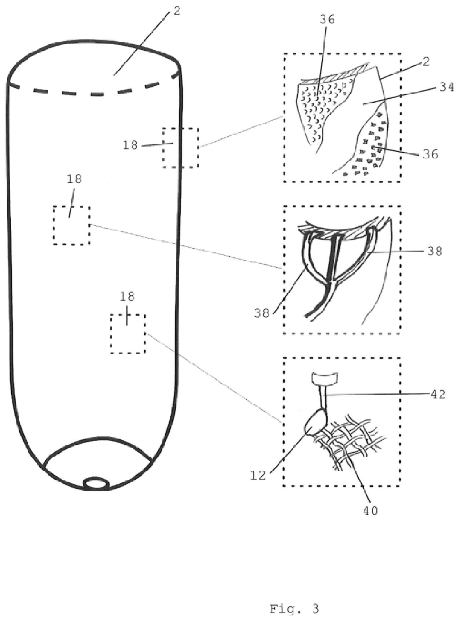

Figur 3 zeigt den Prothesenliner 2 mit drei schematischen Positionskästchen 18, die wieder nur beispielhaft für Positionen unterschiedlicher Elemente stehen können.

Auf der rechten Seite sind verschiedene Strukturierungen angegeben. Im obersten Kästchen ist eine Außenseite des Prothesenliners 2 dargestellt, die Bereiche 34, 36 mit unterschiedlicher Haptik aufweist. Während der mittlere Bereich 34 eine glatte Oberfläche aufweist, ist die Oberfläche des Prothesenliners 2 in den Randbereichen 36 strukturiert ausgebildet.

Im Kästchen darunter sind Evakuierungskanäle 38 dargestellt, die rillenartig in die Seitenwand des Prothesenliners 2 während des additiven Fertigungsverfahrens eingebracht wurden. Durch diese kann ein Unterdruck, der beim angelegten Zustand des Prothesenliners 2 zwischen dem Prothesenliner und einem nicht dargestellten Prothesenschaft entsteht, evakuiert werden.

Im untersten Bereich der Figur 3 ist eine Textillage 40 dargestellt, auf die mittels einer Austrittsdüse 42 während des additiven Fertigungsverfahrens das Fertigungsmaterial 12 aufgebracht wird.

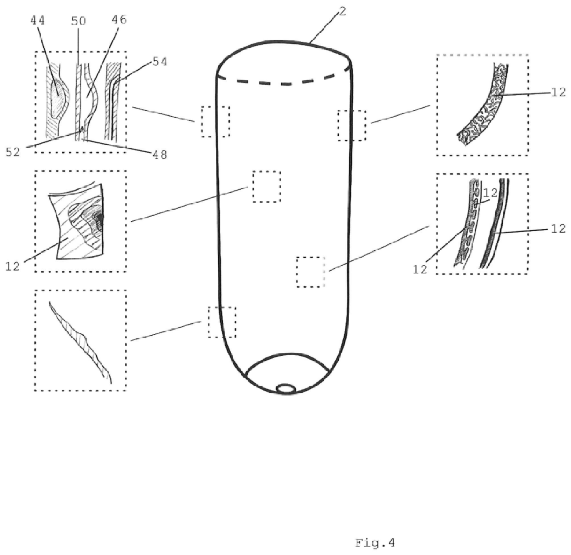

Figur 4 hingegen zeigt den Prothesenliner 2 mit schematischen Darstellungen unterschiedlicher Materialstrukturen. Im Bereich oben links der Figur 4 sind unterschiedliche Arten von Hohlräumen dargestellt, die verschiedene Funktionen aufweisen können. Ganz links ist ein abgeschlossenes Volumen 44 dargestellt, das als Polsterkissen wirkt. Im mittleren Bereich hingegen ist ein Volumenreservoir 46 dargestellt, das über einen Zulauf 48 und einen Ablauf 50 verfügt. Im Zulauf 48 befindet sich ein Flatterventil 52, das als Einwegeventil wirkt. Das Volumenreservoir 46 mit Zulauf 48 und Ablauf 50 kann beispielsweise Teil eines Hydraulik- oder eines Pneumatiksystems sein. Sowohl das Volumenreservoir 46 als auch der Zulauf 48, der Ablauf 50 und das Einwegeventil 52 können während des additiven Fertigungsverfahrens aus dem Fertigungsmaterial 12 hergestellt werden. Im rechten Bereich befindet sich ein Kanal 54, der beispielsweise für Kühlungszwecke verwendet werden kann.

Im Feld darunter ist schematisch dargestellt, dass unterschiedliche Bereiche des Prothesenliners 2, die alle aus einem Fertigungsmaterial 12 hergestellt sein können, unterschiedliche Shore-Härtegrade aufweisen können. Im untersten Bereich ist eine Seitenwand des Prothesenliners 2 im Querschnitt dargestellt. Man erkennt die unterschiedlichen Dicken, die der Liner an unterschiedlichen Stellen aufweisen kann. Die unterschiedlichen Dicken können mit dem additiven Fertigungsverfahren kontinuierlich oder in diskreten Schritten erzeugt werden.

Im oberen Feld auf der rechten Seite der Figur 4 ist schematisch dargestellt, dass das Fertigungsmaterial 12 beispielsweise in Form eines Schaumes, beispielweise eines Silikonschaumes, ausgeführt werden kann. Im Feld darunter werden sogenannte Hybridmaterialien dargestellt, die aus unterschiedlichen Fertigungsmaterialien 12 zusammengesetzt sind. Dabei kann es sich wie im linken Abschnitt um eine formschlüssige Verbindung zwischen den einzelnen Fertigungsmaterialien 12 handeln, während im rechten Teil mehrere Lagen unterschiedlicher Fertigungsmaterialien verwendet werden, die stoffschlüssig miteinander verbunden sind. Mit den Verfahren gemäß den Ausführungsbeispielen der vorliegenden Erfindung können derartige Hybridmaterialien in einem einzigen Fertigungsschritt, nämlich dem additiven Fertigungsverfahren, hergestellt werden.

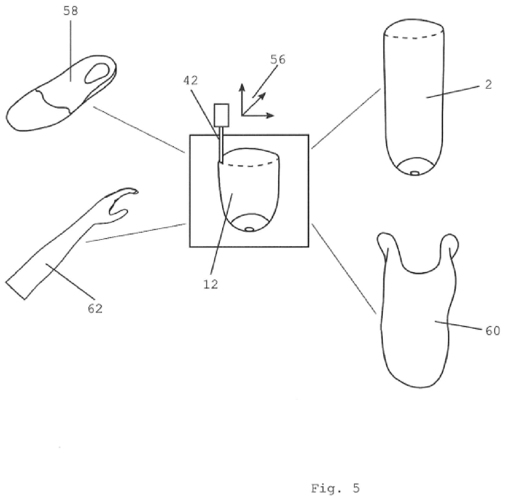

Figur 5 zeigt in der Mitte schematisch ein additives Fertigungsverfahren, wobei es sich im gezeigten Ausführungsbeispiel um das vom MIT entwickelte Rapid-Liquid-Printing-Verfahren handelt. Durch eine Austrittsdüse 42, die, wie die Pfeile 56 andeuten, in allen drei Raumrichtungen frei beweglich ist, wird das Fertigungsmaterial 12 ausgestoßen und an der gewünschten Position in das Stützmaterial eingebracht. Durch die um das zentrale Feld in der Figur 5 angeordneten Produkte wird die Vielfalt der möglichen orthopädietechnischen Einrichtungen, die auf diese Weise hergestellt werden können, dargestellt. Es handelt sich beispielsweise um eine Einlegesohle 58, den Prothesenliner 2, einen Prothesenschaft 60 und einen Prothesenhandschuh 62, wie er beispielsweise zur Umhüllung einer Prothesenhand verwendet wird.

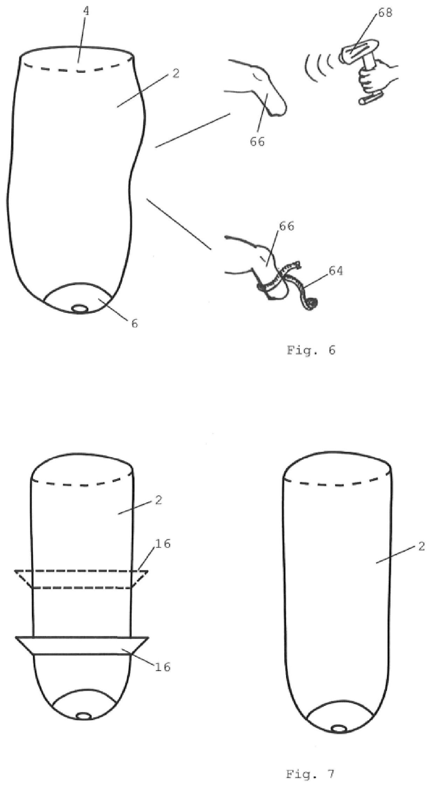

Figur 6 zeit schematisch, dass ein individuell geformter Prothesenliner 2 mit proximaler Öffnung 4 sowie distaler Linerkappe 6 hergestellt werden kann. Dies kann einerseits geschehen, indem beispielsweise mittels eines Maßbandes 64 oder einer anderen klassischen Messmethode ein Amputationsstumpf 66 vermessen wird. Alternativ oder zusätzlich dazu kann, wie im oberen Teil der Figur 6 dargestellt, auch der Amputationsstumpf 66 berührungslos mittels eines Scanners 68 vermessen werden. Unabhängig von der verwendeten Messmethode werden die ermittelten Messdaten einer elektrischen und/oder einer elektronischen Steuerung zur Verfügung gestellt, die die Fertigungsvorrichtung, die zum additiven Fertigungsverfahren verwendet wird, steuern.

Figur 7 zeigt im rechten Bereich den bereits bekannten Prothesenliner 2, der eine Standardgröße und Standardform aufweist. Im linken Teil ist ebenfalls der Prothesenliner 2 dargestellt, an dem nun jedoch eine Dichtlippe 16 angeordnet wurde. Durch die gestrichelt dargestellte Dichtlippe 16 wird schematisch dargestellt, dass diese Dichtlippe in unterschiedlichen Positionen, individuell verschieden, an dem Grundprothesenliner 2 angeordnet werden kann.

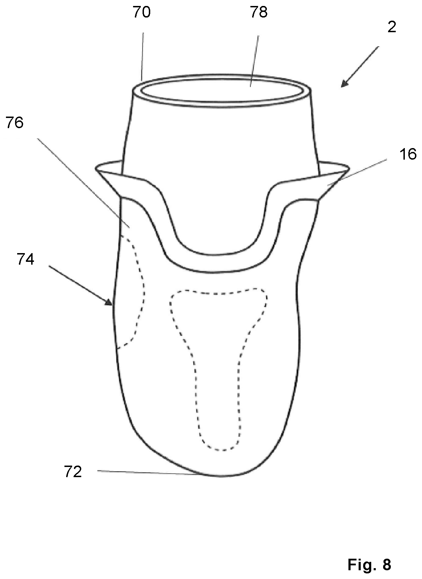

Figur 8 zeigt in einer Einzeldarstellung einen Prothesenliner 2 mit einem proximalen Rand 70 und einem distalen Endbereich 72. Der distale Endbereich 72 ist geschlossen ausgebildet, der proximale Rand 70 umgibt eine Einstiegsöffnung umfänglich. Der Prothesenliner 2 weist einen Grundkörper 74 mit einer Außenseite 76 und einer Innenseite 78 auf. Der Grundkörper 74 ist flexibel und bevorzugt elastisch, zumindest in Umfangsrichtung ausgebildet. Die Innenseite 78 des Grundkörpers 74 besteht vorzugsweise aus einem haftenden Polymer, beispielsweise Silikon. Alternativ dazu kann die Innenseite 78 mit einer haftenden Beschichtung vollständig und teilweise beschichtet sein. Die Beschichtung kann beispielsweise aus einem Silikon oder einem anderem, an der Haut haftenden Polymer ausgebildet sein. Die Außenseite 76 des Grundkörpers 74 kann ebenfalls aus einem Elastomer bestehen oder zumindest teilweise mit einem Elastomer beschichtet sein. Ebenfalls ist es möglich, dass an der Außenseite 76 ein Textil aufgebracht ist, um eine Vergleichmäßigung des Druckes in einem Zwischenraum zwischen dem Prothesenliner 2 und einem nicht dargestellten Prothesenschaft herzustellen. Alternativ oder ergänzend können an der Außenseite 76 Erhebungen oder Kanäle angeordnet sein, beispielsweise ausgebildet oder eingebracht bzw. aufgebracht, um strömungstechnische Verbindungen über die gesamte Längserstreckung, also von distal nach proximal, sowie um den Umfang herum zu ermöglichen.

An dem Grundkörper 74 ist eine Dichtlippe 16 angeordnet, die eine Abdichtung zwischen dem proximalen und dem distalen Bereich des Prothesenliners 2 im eingeführten Zustand in einen nicht dargestellten Prothesenschaft ausbildet. Die Dichtlippe 16 kann aus einem luftundurchlässigen Material hergestellt oder entsprechend beschichtet sein, dass kein Luftdurchtritt durch die Dichtlippe 16 erfolgt. Die Dichtlippe 16 kann beispielsweise aus einem Silikon oder einem Polymer bestehen oder mit einem solchen Werkstoff beschichtet sein. Die Dichtlippe 16 wird bevorzugt einstückig mit dem Grundkörper 74 im Rahmen des wenigstens einen additiven Herstellverfahrens, beispielsweise über das Rapid-Liquid-Printing-Verfahren hergestellt. Der Bereich distal der Dichtlippe 16 an der Außenseite 76 des Grundkörpers 74 kann mit einer strukturierten Oberfläche versehen sein, die eine Druckverteilung in voneinander beabstandeten Bereichen ermöglichen. Die Strukturierung kann beispielsweise als Textilmaterial, das aufgeklebt oder aufkaschiert werden kann, oder über Kanäle und/oder Erhebungen an der Außenseite 76 erfolgen.

Die Dichtlippe 16 steht radial von dem Grundkörper 74 ab und ist bevorzugt elastisch ausgebildet, sodass die Dichtlippe 16 mit ihrer Außenseite, die von dem Grundkörper 74 weg weist, an dem Prothesenschaft anliegt und dagegen drückt. In dem dargestellten Ausführungsbeispiel ist die Dichtlippe 16 nicht senkrecht von der Außenseite 76 des Grundkörpers 74 abstehend ausgebildet, sondern geneigt ausgebildet oder angeordnet. Die Innenseite der Dichtlippe 16, die dem Grundkörper 74 zugewandt ist, schließt einen spitzen Winkel zwischen sich ein. Grundsätzlich ist es auch möglich, eine umgekehrt gerichtete Orientierung vorzusehen oder die Dichtlippe 16 senkrecht abstehen zu lassen. Beim Einführen des Prothesenliners 2 in einen Prothesenschaft wird dann die Dichtlippe 16 in der Regel umgeschlagen, sodass sich eine Orientierung ergibt, bei der die in distale Richtung orientierte Seite der Dichtlippe 16 an der Innenseite des Prothesenschaftes anliegt. Bei einem Unterdruck in dem durch die Dichtlippe 16 abgedichteten Volumen zwischen dem Prothesenschaft und dem distal zu der Dichtlippe 16 befindlichen Bereich gegenüber dem Atmosphärendruck, wird die Dichtlippe 16 gegen die Innenwand des Prothesenschaftes gedrückt, so dass sich ein selbstverstärkender Dichteffekt einstellt.

Der Figur 8 ist zu entnehmen, dass der proximale Rand 70 des Prothesenliners 2 geradlinig oder in einer Ebene angeordnet ausgebildet ist, wobei die Ebene im Wesentlichen senkrecht zu der Längserstreckung des Prothesenliners 2 verläuft.

Davon abweichend verläuft die Dichtlippe 16 nicht in einer gemeinsamen Ebene, insbesondere nicht in einer Ebene parallel oder geneigt zu dem proximalen Rand 70 des Prothesenliners 2, sondern entlang einer Raumkurve, die korrespondierend zu dem Verlauf der Höhenkontur des Prothesenschaftes an seinem proximalen Rand verläuft. In dem dargestellten Ausführungsbeispiel der Figur 8 ist ein Prothesenliner 2 für einen Unterschenkel gezeigt. Der Schienbeinkopf ist in der unterbrochenen Linie angedeutet. Die Dichtlippe 16 verläuft im frontalen Bereich knapp oberhalb des Schienbeinkopfes und erstreckt sich medial und lateral in Richtung auf den proximalen Rand 70. Im rückwärtigen Teil des Prothesenliners kann die Dichtlippe 16 wieder in Distalrichtung abgesenkt verlaufen. Ein solcher Verlauf entspricht dem Verlauf des proximalen Randes eines Unterschenkelschaftes, der im frontalen Schienbeinbereich und im Kniekehlenbereich tiefer, also weiter in Richtung distal verläuft als medial-lateral. Medial und lateral des Kniegelenkes können Prothesenschaftbereiche weiter in Proximalrichtung verlaufend angeordnet sein, um eine erhöhte seitliche Stabilität und eine verbesserte Anlage des Unterschenkelschaftes an dem Stumpf zu erreichen.

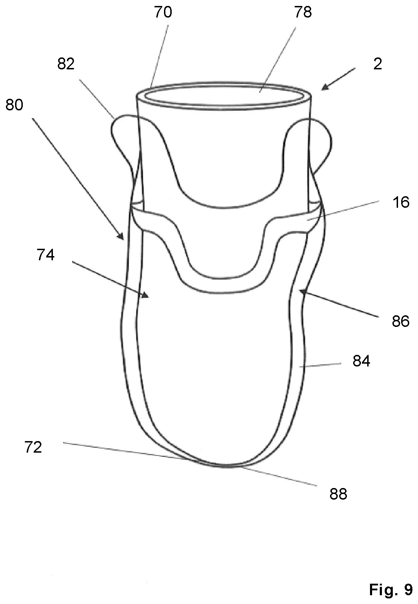

In Figur 9 ist in einer schematischen Darstellung der Prothesenliner 2 gemäß Figur 8 in einem angezogenen Zustand gezeigt. Der Prothesenliner 2 ist an dem nicht dargestellten Stumpf angelegt und in einen Prothesenschaft 80 eingeführt. Der Prothesenschaft 80 weist einen proximalen Rand 82 auf, der nicht in einer flachen Ebene liegt, sondern eine Raumkurve beschreibt. Der Prothesenschaft 80 weist medial und lateral hochgezogene Bereiche auf, die sich weiter in Proximalrichtung erstrecken als die Bereiche, die frontal und im Bereich der Kniekehle angeordnet sind. Frontal ist ein Ausschnitt zu erkennen, der es ermöglicht, dass die Kniescheibe beweglich ist. Im rückwärtigen Bereich der Kniekehle ist ein korrespondierender Ausschnitt oder eine korrespondierende Absenkung ausgebildet, um ein Einbeugen des Beines zu ermöglichen, ohne dass der Prothesenschaft mit seinem dorsalen Bereich zwischen dem rückwärtigen Oberschenkel und dem Wadenbereich eingeklemmt wird.

Der Prothesenliner 2 ist vollständig in einen Aufnahmeraum 84 des Prothesenschaftes 80 eingeführt, das heißt, dass das distale Ende 72 des Prothesenliners 2 im Bereich des distalen Endes 88 des Prothesenschaftes 80 befindlich ist, gegebenenfalls darauf aufliegt oder leicht beabstandet dazu, beispielsweise über ein Polster, daran angeordnet ist. Die Dichtlippe 16 liegt an der Innenwand des Prothesenschaftes 80 an und dichtet ein Volumen 86 zwischen der Innenwand des Prothesenschaftes 80 und der Außenwand 76 des Prothesenliners 2 distal zu der Dichtlippe 16 ab. Das Volumen 86 wird beispielsweise durch eine Pumpbewegung beim Gehen durch ein Auslassventil oder durch eine motorisch angetriebene Pumpe evakuiert, also auf ein Druckniveau gebracht, das unterhalb des Atmosphärendruckes liegt.

Der Figur 9 ist zu entnehmen, dass der Dichtlippenverlauf dem Verlauf des proximalen Randes 82 des Prothesenschaftes 80 entspricht oder diesem folgt und lediglich in Distalrichtung versetzt an der Außenseite des Grundkörpers 74 befindlich ist oder angeordnet ist. Idealerweise verläuft die Dichtlippe 16 in geringstmöglicher Entfernung zu dem proximalen Rand 82 des Prothesenschaftes 80. Insbesondere der Höhenverlauf oder die Höhenkontur, also der Verlauf der Dichtlippe 16 um den Umfang des Grundkörpers 74 in Proximal-Distal-Richtung, entspricht dem Höhenverlauf des proximalen Randes 82 des Prothesenschaftes. Geringe Abweichungen können möglich sein, insbesondere kann der Dichtlippenverlauf in einem Bereich festgelegt werden, der im Wesentlichen parallel zu dem Verlauf der Höhenkontur des proximalen Randes 82 des Prothesenschaftes 80 entspricht, wobei die proximale und distale Grenze des Bereiches zu dem Höhenkonturverlauf des proximalen Randes 82 korrespondierend ausgebildet ist.

Auch die Kontur in Umfangsrichtung, also die Kontur des Innenumfanges des Prothesenschaftes 80 im Bereich der Anlage der Dichtlippe 16 kann erfasst werden. Die Kontur des äußeren Umfanges der Dichtlippe 16 kann dann korrespondierend zu dem Verlauf der Umfangskontur im Bereich der Anlage des äußeren Dichtlippenrandes an der Innenseite des Prothesenschaftes 80 ausgebildet sein, versehen mit einem Zuschlag, sodass die Dichtlippe 16 an der Innenseite des Prothesenschaftes 80 mit einer leichten Vorspannung aufgrund der Rückstellkräfte bei einer Verformung nach dem Einführen des Prothesenliners 2 in dem Prothesenschaft 80 anliegen kann.

Alternativ zu einer Ausgestaltung des Prothesenschaftes 80 als Unterschenkelschaft mit Erhöhungen auf der medialen und lateralen Seite kann beispielsweise eine Ausgestaltung als Oberschenkelschaft eine nur einseitige Erhöhung lateral beinhalten, die ungefähr bis zur Drehachse des Hüftgelenkes reicht. Auf der medialen Seite des Oberschenkels ist dementsprechend ein in Distalrichtung versetzter Ausschnitt ausgebildet, sodass sich ein entsprechender Dichtlippenverlauf bei einem Oberschenkelliner einstellt.

Zur Herstellung eines solchen Liners 2 wird zunächst die Höhenkontur des Prothesenschaftes 80, der in der Regel individuell angefertigt wird, erfasst. Dazu wird auch die Höhe des Prothesenschaftes 80 erfasst, also der Abstand von dem proximalen Rand 82 bis zu dem distalen Ende 12 auf der Innenseite des Prothesenschaftes 80 über den Umfang des Stumpfes. Die Form und die Abmessungen können bevorzugt optisch erfasst werden, beispielsweise durch Bildaufnahmen und Bildauswertung, alternative Erfassungsdaten wie ein Abtasten oder ein Abfahren mit Messwertaufnehmern kann ebenfalls erfolgen.

Auf der Grundlage des erfassten Verlaufes der Höhenkontur des proximalen Randes 82 wird dann festgelegt, wo die Dichtlippe 16 an der Innenseite des Prothesenschaftes anliegen soll und damit wo die Dichtlippe an der Außenseite 76 des Grundkörpers 74 des Prothesenliners 2 anzuordnen ist. Die erfassten Daten werden dazu benutzt, um ein 3D-Datenmodell zu erstellen. Anhand des Datenmodells des Prothesenschaftes 80 wird der Liner 2 konstruiert, beispielsweise mit einem Standardgrundkörper 74 und einen individuellen Dichtlippenverlauf der Dichtlippe 16, der an dem Verlauf des proximalen Randes 82 des Prothesenschaftes 80 orientiert ist. Die Form des Prothesenliners 2 mit dem angepassten Dichtlippenverlauf wird ebenfalls als 3D-Datenmodell errechnet. Anhand des 3D-Datenmodells werden Fertigungsdaten erzeugt, anhand derer mit dem wenigstens einen additiven Fertigungsverfahren der Prothesenliner 2 mit dem Dichtlippenverlauf korrespondierend zu dem Verlauf des proximalen Randes 82 des Prothesenliners gefertigt wird.

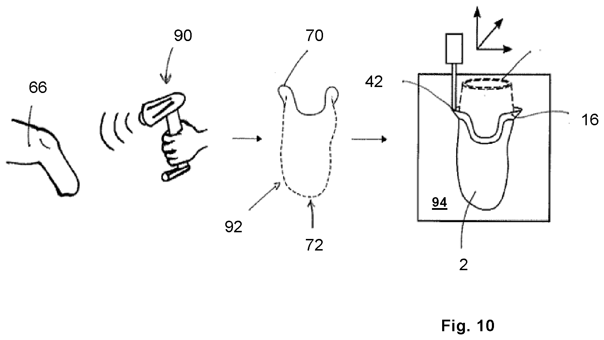

In der Figur 10 ist ein möglicher Ablauf eines Verfahrens zum Herstellen eines Prothesenliners 2 dargestellt. Von einem Amputationsstumpf 66, vorliegend einem Unterschenkelstumpf, wird über ein optisches Erfassungsgerät 90 die äußere Kontur des Amputationsstumpfes 66 aufgenommen, beispielsweise gescannt. Von dem Amputationsstumpf 66 wird ein 3D-Modell erstellt und in einem nicht dargestellten Rechner aufbereitet. Anhand des 3D-Modells wird ein Datensatz 92 errechnet, der zumindest im Wesentlichen die Form des späteren Prothesenliners 2 repräsentiert. Der Datensatz 92 legt neben dem Dichtlippenverlauf auch die äußere Kontur des Prothesenliners 2 fest, insbesondere auch den distalen Endbereich 72 sowie die Materialstärke des Prothesenliners 2. Über den Datensatz 92 ist es möglich, Verstärkungen, Materialschwächungen sowie die Verwendung unterschiedlicher Materialien zu definieren, die dann während des Fertigungsverfahrens verwendet oder eingearbeitet werden. Anhand des Datensatzes 92 kann der tatsächliche Prothesenliner 2 gefertigt werden. Der proximale Rand 70 beziehungsweise der Verlauf des proximalen Randes 70 des tatsächlichen Prothesenschaftes 2 im Raum ist in dem Ausführungsbeispiel noch nicht als im Datensatz 92 festgelegt eingezeichnet. Die übrige Kontur des Prothesenliners 2 ist durch die unterbrochene Linie angedeutet. Anhand des Datensatzes 92 oder der Grunddaten des Scanns kann ein Datensatz für den Prothesenschaft 80 erstellt werden, der die Grundlage für dessen Fertigung bildet, beispielsweise in einem additiven Fertigungsverfahren. Der Dichtlippenverlauf im Raum ist als Konturlinie definiert und kann als Referenz für den Verlauf der Kontur des proximalen Randes 82 des Prothesenschaftes 80 dienen. Der Dichtlippenverlauf der Dichtlippe 16 an der Außenseite eines noch zu fertigenden Prothesenliners 2 kann also zuerst bestimmt werden, anschließend wird der Prothesenschaft 80 gestaltet. Es besteht umgekehrt die Möglichkeit, den Dichtlippenverlauf an einer bereits festgelegten Kontur des proximalen Randes 82 eines virtuellen oder bereits bestehenden Prothesenschaftes 80 anzupassen.

Anhand des Datensatzes 92 wird mit einem additiven Fertigungsverfahren der Prothesenliner 2 gefertigt. In dem dargestellten Ausführungsbeispiel erfolgt die Herstellung nach dem sogenannten Rapid-Liquid-Printing-Verfahren, bei dem ein Stützmaterial 94 in einem Tank oder Vorratsbehälter angeordnet ist. Über eine Austrittsdüse 42, die dreidimensional im Raum verfahrbar ist, wird das Material des Prothesenliners 2 in das Stützmaterial 94 eingebracht und der Prothesenliner 2 additiv gefertigt. Die gestrichelte Linie deutet die proximale Endkontur des Prothesenliners 2 an, die in dem dargestellten Ausführungsbeispiel geradlinig ist. Die proximale Endkontur oder der proximale Rand 70 des Prothesenliners 2 kann auch korrespondierend zu dem Verlauf der Dichtlippe 16 oder korrespondierend zu dem proximalen Rand 82 des Prothesenschaftes 80 verlaufen.

### Bezugszeichenliste

- 2: Prothesenliner
- 4: Öffnung
- 6: Linerkappe
- 8: Kissen
- 10: Elektrode
- 12: Fertigungsmaterial
- 14: Zuführung
- 16: Dichtlippe
- 18: Positionskästchen
- 20: Wulst

- 22: Anschlusselement
- 24: Kabel
- 26: elektrischer Leiter
- 28: Markierungslinie
- 30: Designelement

- 32: Verschleißindikator
- 34: mittlerer Bereich
- 36: Randbereich
- 38: Evakuierungskanal
- 40: Textillage

- 42: Austrittsdüse
- 44: abgeschlossenes Volumen
- 46: Volumenreservoir
- 48: Zulauf
- 50: Ablauf

- 52: Flatterventil
- 54: Kanal
- 56: Pfeil
- 58: Einlegesohle
- 60: Prothesenschaft

- 62: Prothesenhandschuh
- 64: Maßband
- 66: Amputationsstumpf
- 68: Scanner
- 70: proximaler Rand

- 72: distaler Endbereich
- 74: Grundkörper
- 76: Außenseite
- 78: Innenseite
- 80: Prothesenschaft

- 82: proximaler Rand
- 84: Aufnahmeraum
- 86: Volumen
- 88: distales Ende
- 90: optisches Erfassungsgerät

- 92: Datensatz
- 94: Stützmaterial

## Patentansprüche

1. Verfahren zum Herstellen eines Prothesenliners (2), der wie ein Strumpf über einen Amputationsstumpf gezogen werden kann, **dadurch gekennzeichnet, dass** die orthopädietechnische Einrichtung zumindest teilweise mittels eines additiven Fertigungsverfahrens aus wenigstens einem Fertigungsmaterial (12) hergestellt wird, das in einem fließfähigen Zustand in ein Stützmaterial eingebracht wird und danach aushärtet, wobei das Fertigungsmaterial (12) aus wenigstens zwei Komponenten zusammengesetzt wird, wobei vorzugsweise ein Mischungsverhältnis der wenigstens zwei Komponenten während des additiven Fertigungsverfahrens einstellbar ist.

2. Verfahren nach Anspruch 1, **dadurch gekennzeichnet, dass** das Fertigungsmaterial (12) nach dem Aushärten eine Shore-Härte aufweist, deren Wert von dem Mischungsverhältnis abhängt.

3. Verfahren nach Anspruch 1 oder 2, **dadurch gekennzeichnet, dass** in dem additiven Fertigungsverfahren wenigstens zwei unterschiedliche Fertigungsmaterialien (12) verwendet werden, die sich nach dem Aushärten in ihrer Härte und/oder in ihrer Elastizität unterscheiden

4. Verfahren nach einem der vorstehenden Ansprüche, **dadurch gekennzeichnet, dass** der Prothesenliner ein Prothesenliner für Unterschenkelamputierte ist, der im Bereich der Kniekehle deutlich elastischer ausgeführt ist, als dies in anderen Bereichen der Fall ist.

5. Verfahren nach Anspruch 4, **dadurch gekennzeichnet, dass** der Bereich der Kniekehle mit einem besonders elastischen Fertigungsmaterial hergestellt wird.

6. Verfahren nach einem der vorstehenden Ansprüche, **dadurch gekennzeichnet, dass** die beiden unterschiedlichen Fertigungsmaterialien (12) gleichzeitig verwendet werden.

7. Verfahren nach einem der vorstehenden Ansprüche, **dadurch gekennzeichnet, dass** die Teile, an denen ein Knochen sehr nah an der Außenseite des Amputationsstumpfes positioniert ist und die einer besonderen Polsterung bedürfen, mit einem besonders weichen und polsternden Material überdeckt werden.

8. Verfahren nach einem der vorstehenden Ansprüche, **dadurch gekennzeichnet, dass** mit dem additiven Fertigungsverfahren eine orthopädietechnische Einrichtung mit wenigstens einem Hohlraum erzeugt wird.

9. Verfahren nach Anspruch 8, **dadurch gekennzeichnet, dass** der wenigstens eine Hohlraum während des additiven Fertigungsverfahrens mit wenigsten einem Füllmaterial zumindest teilweise, bevorzugt vollständig, gefüllt wird, wobei bevorzugt wenigstens zwei Hohlräume mit unterschiedlichen Füllmaterialien befüllt werden.

10. Verfahren nach einem der vorstehenden Ansprüche, **dadurch gekennzeichnet, dass** mittels des additiven Fertigungsverfahrens aus dem wenigstens einen Fertigungsmaterial (12) wenigstens ein pneumatisches und/oder wenigstens ein hydraulisches Element, vorzugsweise wenigstens ein Volumenreservoir (46), wenigstens eine Dichtlippe (16), wenigstens ein Ventil und/oder wenigstens eine Pumpe hergestellt wird, das bevorzugt einstückig mit einem anderen Bauteil der orthopädietechnischen Einrichtung hergestellt wird.

11. Verfahren nach einem der vorstehenden Ansprüche, **dadurch gekennzeichnet, dass** Messdaten von einem Patienten erfasst und einer elektrischen und/oder elektronischen Steuerung zur Verfügung gestellt werden, die eingerichtet ist, zumindest auch auf der Grundlage der Messdaten das additive Fertigungsverfahren zu steuern.

12. Verfahren nach einem der vorstehenden Ansprüche, **dadurch gekennzeichnet, dass** die orthopädietechnische Einrichtung ein Prothesenliner (2) zum Einsatz in einem Prothesenschaft (80) ist, wobei der Prothesenschaft (80) einen Aufnahmeraum (84) mit einem distalen Ende (88) und einem proximalen Rand (82) aufweist, wobei das Verfahren folgende Schritte aufweist:
a) Festlegen eines Dichtlippenverlaufes an einer Außenseite (76) des Prothesenliners (2) korrespondierend zu dem Verlauf einer Höhenkontur des Prothesenschaftes (80) oder anhand vorhandener, bekannter anatomischer Gegebenheiten eines Amputationsstumpfes und
b) Anordnen einer Dichtlippe (16) an der Außenseite (76) des Prothesenliners (2) entlang des festgelegten Dichtlippenverlaufes mittels des wenigstens einen additiven Fertigungsverfahrens.

13. Verfahren nach Anspruch 12, **dadurch gekennzeichnet, dass** die Dichtlippe (16) in distaler Richtung zu dem proximalen Rand (82) des Prothesenschaftes (80) versetzt, insbesondere äquidistant an dem Prothesenliner (2) angeordnet ist.

14. Verfahren nach Anspruch 12 oder 13, **dadurch gekennzeichnet, dass** die Höhenkontur des Prothesenschaftes (80) optisch erfasst wird, ein digitales 3D-Modell erstellt wird und der Dichtlippenverlauf in Abhängigkeit von der erfassten Höhenkontur festgelegt wird.

15. Orthopädietechnische Einrichtung, hergestellt oder herstellbar nach einem Verfahren gemäß einem der vorstehenden Ansprüche.
